# EUROPÄISCHE PATENTSCHRIFT

(11) **EP 0 966 420 B1**
(45) Veröffentlichungstag und Bekanntmachung des Hinweises auf die Patenterteilung: **08.12.2004**
(21) Anmeldenummer: 98912323.7
(22) Anmeldetag: 18.02.1998
(51) Int. Cl.: C07C 49/747, C07C 45/45, C07C 69/013, C07C 69/96, C07C 329/06, A01N 35/06, A01N 37/02, C07C 69/757, C07C 69/738, C07C 62/38, C07C 59/84

(54) **2-ARYLCYCLOPENTAN-1,3-DIONE**
2-ARYLCYCLOPENTANE-1,3-DIONES
2-ARYLCYCLOPENTAN-1,3-DIONES

(30) Priorität: 03.03.1997 DE 19708607
(43) Veröffentlichungstag der Anmeldung: 29.12.1999
(73) Patentinhaber: Bayer CropScience AG, 40789 Monheim (DE)
(72) Erfinder: ERDELEN, Christoph, D-42799 Leichlingen (DE); WACHENDORFF-NEUMANN, Ulrike, D-56566 Neuwied (DE); FISCHER, Reiner, D-40789 Monheim (DE); GRAFF, Alan, D-51061 Köln (DE); MENCKE, Norbert, D-51381 Leverkusen (DE); TURBERG, Andreas, D-40699 Erkrath (DE)
(86) Internationale Anmeldenummer: PCT/EP1998/000916
(87) Internationale Veröffentlichungsnummer: WO 1998/039281

(56) Entgegenhaltungen:
- DE-A- 19 518 962
- US-A- 4 283 348
- US-A- 4 338 122
- US-A- 4 436 666
- US-A- 4 526 723
- US-A- 4 551 547

## Beschreibung

Die vorliegende Erfindung betrifft neue 2-Aryl-3-hydroxy-Δ²-cyclopenten-1-on-Derivate, Verfahren zu ihrer Herstellung und ihre Verwendung als Herbizide und Schädlingsbekämpfungsmittel.

Es ist bekannt, daß bestimmte substituierte 2-Arylcyclopentandione herbizide und akarizide Eigenschaften besitzen (vgl. z.B. US 4 283 348; 4 338 122; 4 436 666; 4 526 723; 4 551 547, 4 632 698, WO 96/01798 und WO 96/03366). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-enone aus der Publikation Edwards et al., J. Chem. Soc. S, (1967), 405-9. Eine insektizide oder akarizide Wirkung wird nicht beschrieben.

Die akarizide und insektizide Wirksamkeit und/oder Wirkungsbreite, und die Pflanzenverträglichkeit dieser Verbindungen, insbesondere gegenüber Kulturpflanzen, ist jedoch nicht immer ausreichend.

Es wurden nun neue Verbindungen der Formel (I) 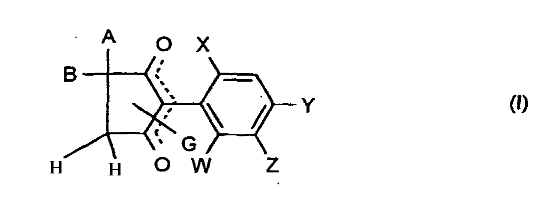 gefunden,
in welcher die Substituenten die nachstehend angegebenen Bedeutungen haben:
W = CH₃; X = CH₃; Y = CH₃; Z = CH₃ oder
W = CH_{3;} X = CH₃; Y = H; Z = Cl oder
W = CH₃; X = CH₃; Y = CH₃; Z = F oder
W = CH₃; X = CH₃; Y = CH₃; Z = Cl oder
W = CH₃; X = CH₃; Y = CH₃; Z = Br oder
W = CH₃; X = CH₃; Y = H; Z = Br oder
W = Cl; X = Cl; Y = H; Z = Br oder
W = Br; X = Br; Y, Z = -(CH₂)₃-oder
W = CH₃; X = OCH₃; Y = H; Z = Br oder
W = CH₃; X = CH₃; Y = CH₃; Z = CH₃ oder
W = Cl; X = Cl; Y = Cl; Z = CH₃ oder
W = Br; X = Br; Y = Br; Z = CH₃ oder
W = H; X = Cl; Y = Cl; Z = Cl oder
W = H; X = CH₃; Y = CH₃, Z = CH₃ oder
W = H, X = CH₃; Y = Cl, Z = CH₃ oder
W = H; X = Br; Y = CH₃; Z = Cl oder
W = H; X = Br; Y = CH₃; Z = CH₃ oder
W = H; X = Cl; Y = Br; Z = CH₃ oder
W = H; X = Cl; Y = Cl; Z = CH₃ oder
W = H; X = CH₃; Y = CH₃; Z = Br oder
W = H; X = Cl; Y, Z = -O-CF₂-O- oder
W = H; X = Br; Y = CH₃; Z = Br oder
W = H; X = CH₃; Y = H; Z = CH₃ oder
W = H; X = Cl; Y = H; Z = NO₂ oder
W = H; X = Br; Y = H; Z = OCH₃,
G = H,

| **A** | **B** |
|---|---|
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -(CH₂)₂-S-(CH₂)₂- | |
| -CH₂-O-(CH₂)₃- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHOi-C₃H₇-(CH₂)₂- | |
| -(CH₂)₂-C(CH₃)₂-(CH₂)₂- | |
| -CH₂-(CHCH₃)₂-(CH₂)₂- | |

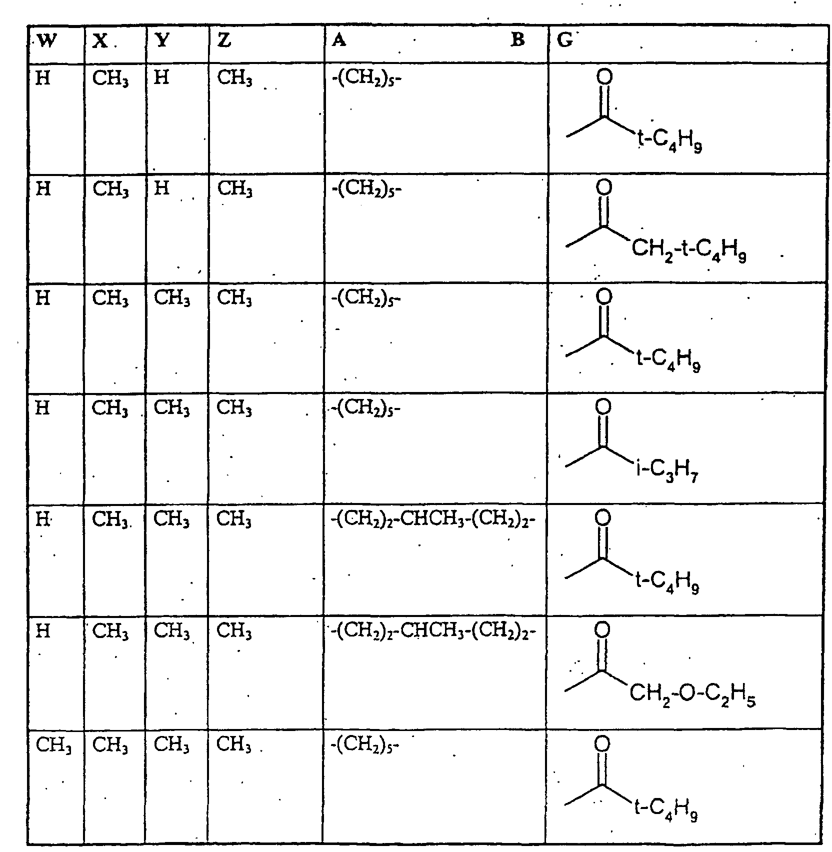

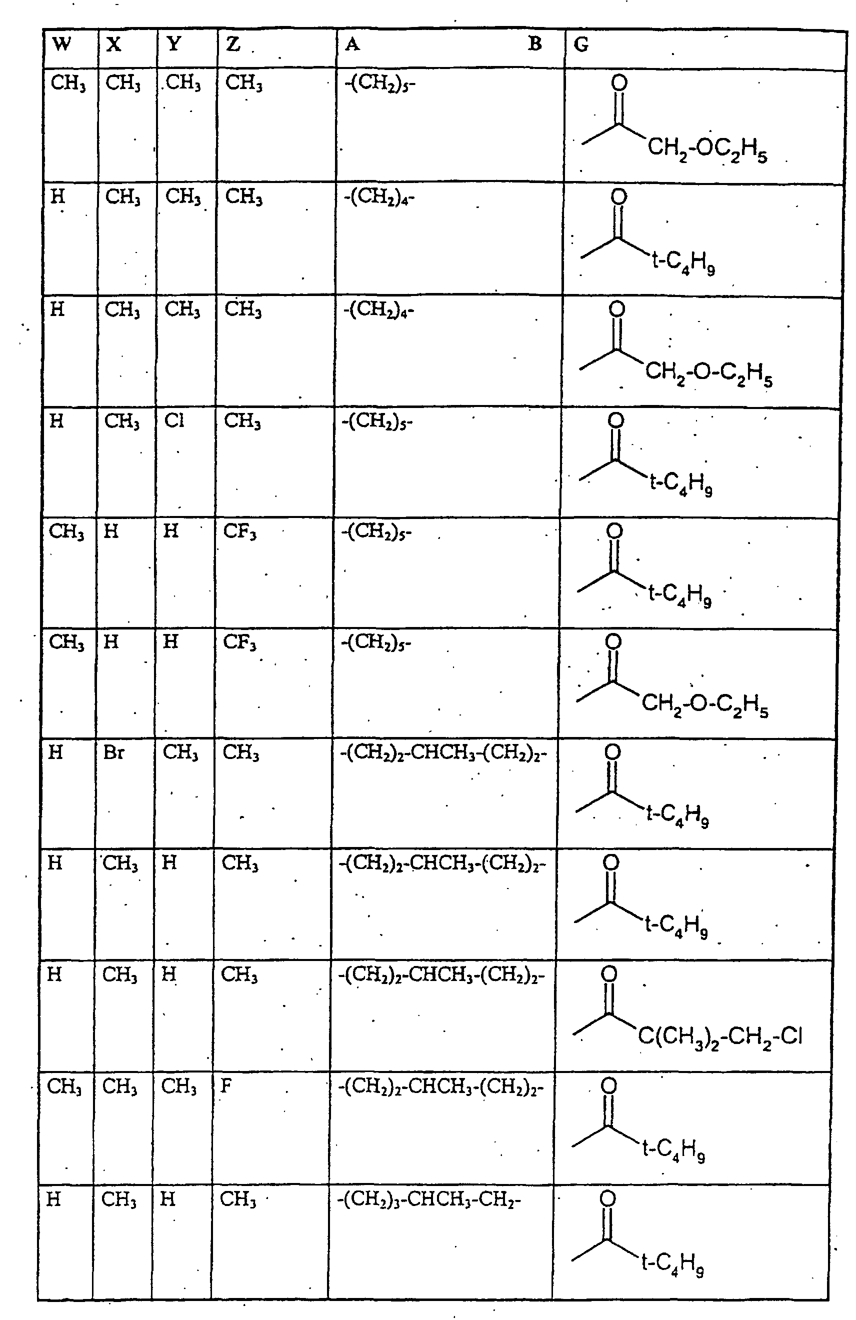

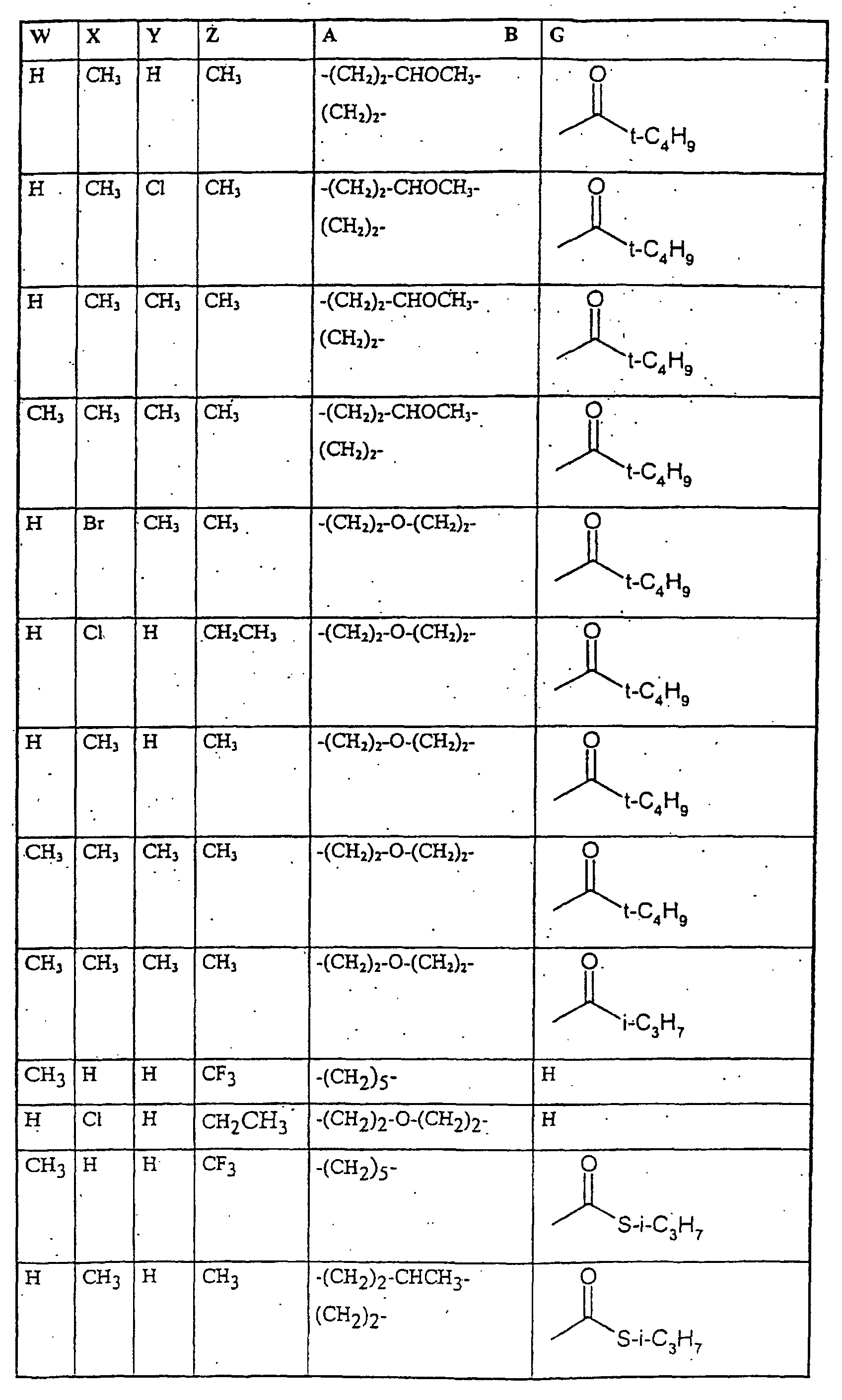

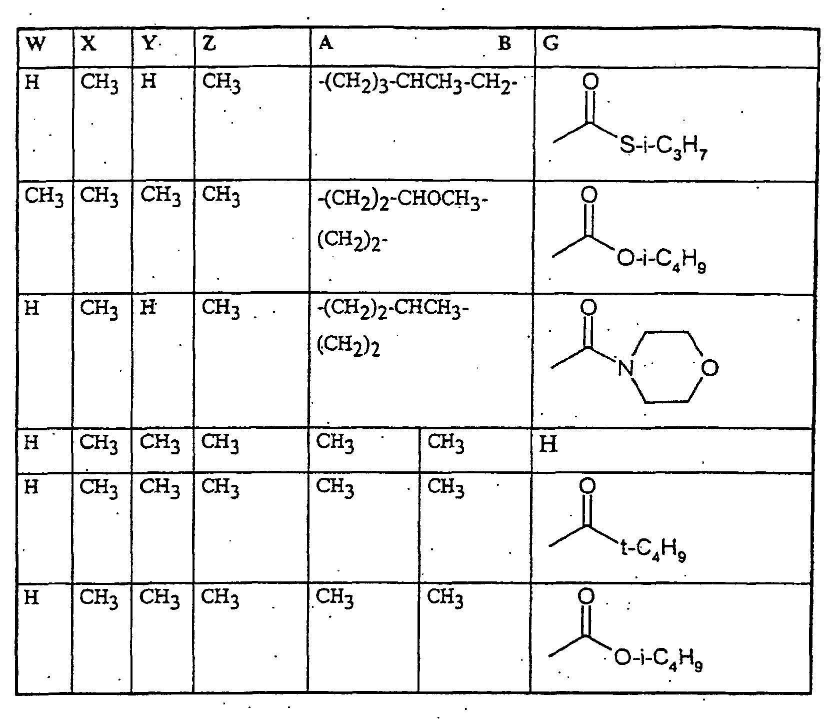

sowie die enantiomerenreinen Formen von Verbindungen der Formel (I).

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Weise durch physikalische Methoden, beispielsweise durch chromatographische Methoden, getrennt werden.

Die Verbindungen der Formel (I) können in Abhängigkeit von der Stellung des Substituenten G in den zwei isomeren Formen der Formeln I-A und I-B vorliegen, 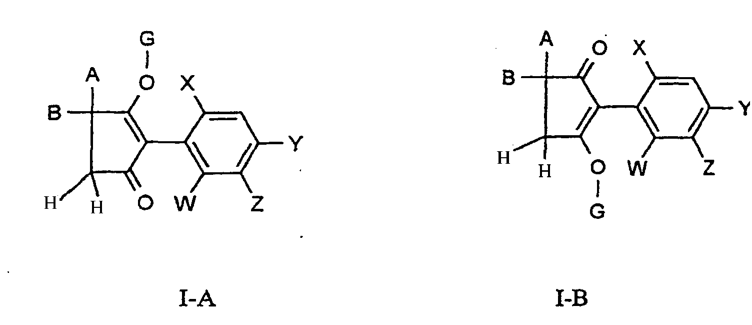 was durch die gestrichelte Linie in der Formel (I) zum Ausdruck gebracht werden soll.

Die Verbindungen der Formeln I-A und I-B können sowohl als Gemische als auch in Form ihrer reinen Isomeren vorliegen. Gemische der Verbindungen der Formeln I-A und I-B lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen in Form der Isomerengemische oder in der jeweils anderen isomeren Form vorliegen können.

Weiterhin wurde gefunden,
(A) daß man Verbindungen der Formel (Ia) 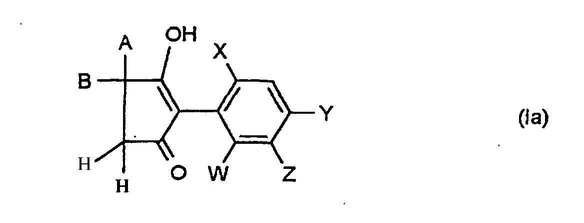 in welcher
   A, B, W, X, Y und Z die oben angegebene Bedeutung haben,
   erhält, wenn man
   Ketocarbonsäureester der Formel (II) 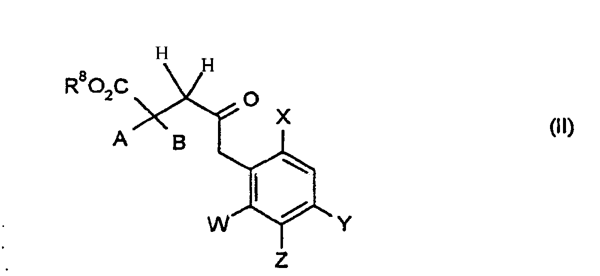 in welcher
   A, B, W, X, Y und Z die oben angegebene Bedeutung haben, und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert und
(B) daß man die Verbindungen der oben gezeigten Formel (Ib), in welcher A, B, R¹, W, X, Y und Z die oben angegebenen Bedeutungen haben, erhält, wenn man Verbindungen der oben gezeigten Formel (Ia), in welcher A, B, W, X, Y und Z die oben angegebenen Bedeutungen haben,
   α) mit Säurehalogeniden der Formel (III) 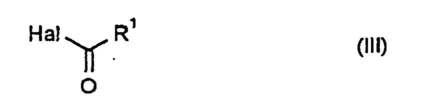 in welcher
      - R¹: die oben angegebene Bedeutung hat und
      - Hal: für Halogen (insbesondere Chlor oder Brom) steht
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(C) daß man die Verbindungen der oben gezeigten Formel (Ic), in welcher A, B, R², W, M, X, Y und Z die oben angegebenen Bedeutungen haben und L für Sauerstoff steht, erhält, wenn man Verbindungen der oben gezeigten Formel (Ia), in welcher A, B, W, X, Y und Z die oben angegebenen Bedeutungen haben,
   mit Chlorameisensäureestern oder Chlorameisensäurethioestern der Formel (V)

   R²-M-CO-Cl (V)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(G) daß man Verbindungen der oben gezeigten Formel (If), in welcher A, B, L, R⁶, R⁷; W, X, Y und Z die oben angegebenen Bedeutungen haben, erhält, wenn man Verbindungen der oben gezeigten Formel (Ia), in welcher A, B, W, X, Y und Z die oben angegebenen Bedeutungen haben, jeweils
   α) mit Isocyanaten oder Isothiocyanaten der Formel (XI)

      R⁶-N=C=L (XI)
   in welcher
   - R⁶ und L: die oben angegebenen Bedeutungen haben,
   gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XII) 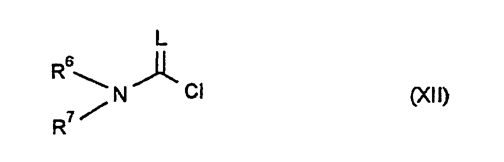
   in welcher
   - L, R⁶ und R⁷: die oben angegebenen Bedeutungen haben,
   gegebenenfalls in Gegenwart eines Verdünnungsmittels und gegebenenfalls in Gegenwart eines Säurebindemittels, umsetzt,

Weiterhin wurde gefunden, daß die neuen Verbindungen der Formel (I) eine sehr gute Wirksamkeit als Schädlingsbekämpfungsmittel, vorzugsweise als Insektizide und Akarizide und mit höheren Aufwandmengen auch als Herbizide aufweisen und darüber hinaus sehr gut pflanzenverträglich, insbesondere gegenüber Kulturpflanzen, sind.

Verwendet man gemäß Verfahren (A) 5-(2,4,6-Trichlor-3-methylphenyl)-2,2-dimethyl-4-oxo-valeriansäure-ethylester, so kann der Verlauf des erfindungsgemäßen Verfahrens durch folgendes Reaktionsschema wiedergegeben werden:

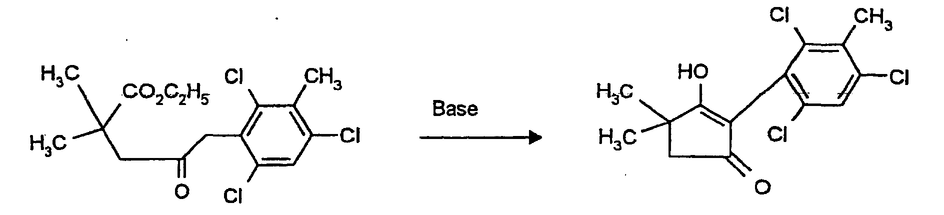

Verwendet man gemäß Verfahren (B) (Variante α) 2-(2,4,6-Trimethyl-3-chlorphenyl)-3-hydroxy-4,4-dimethyl-Δ²-cyclopentenon und Pivaloylchlorid als Ausgangsstoff, so kann der Verlauf des erfindungsgemäßen Verfahrens durch folgendes Reaktionsschema wiedergegeben werden.

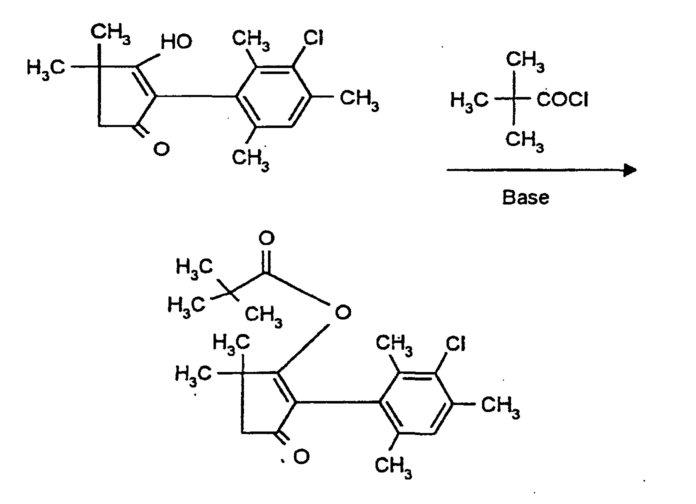

Verwendet man gemäß Verfahren (B) (Variante β) 2-(2,5-Dimethylphenyl)-3-hydroxy-4-methyl-4-phenyl- ²-cyclopentenon und Acetanhydrid als Ausgangsverbindungen, so kann der Verlauf des erfindungsgemäßen Verfahrens durch folgendes Reaktionsschema wiedergegeben werden.

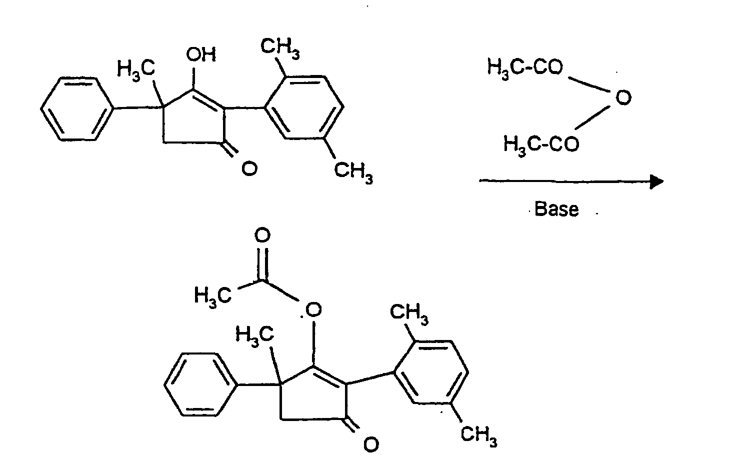

Verwendet man gemäß Verfahren (C) 2-(2,4-Dichlor-5-methylphenyl)-3-hydroxy-4-isopropyl-4-methyl-Δ²-cyclopentenon und Chlorameisensäureethoxyethylester als Ausgangsverbindungen, so kann der Verlauf des erfindungsgemäßen Verfahrens durch folgendes Reaktionsschema wiedergegeben werden.

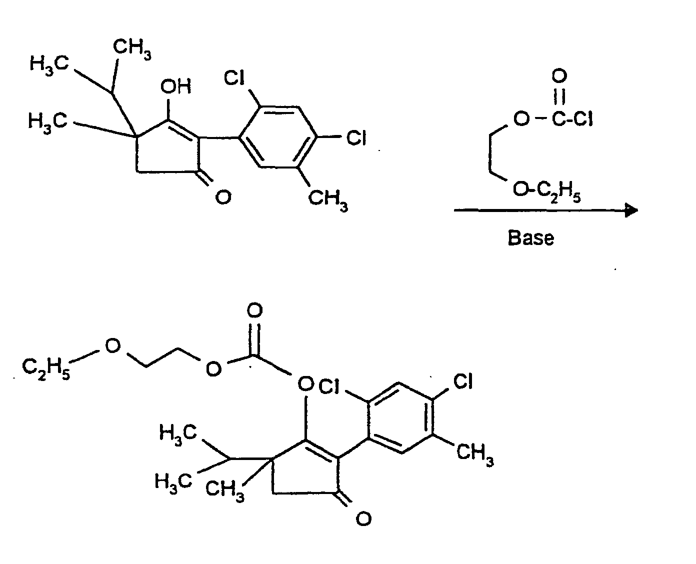

Verwendet man gemäß Verfahren (Gα) 2-(2,6-Dimethyl-3-bromphenyl)-3-hydroxy-4,4-tetramethylen-Δ²-cyclopentenon und Ethylisocyanat als Ausgangsprodukte, so kann der Reaktionsverlauf durch folgendes Reaktionsschema wiedergegeben werden:

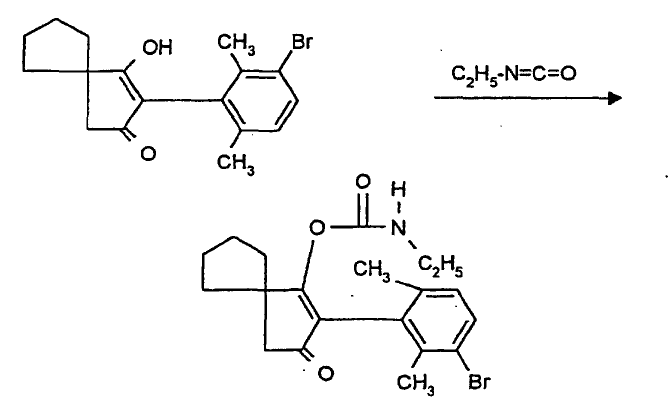

Verwendet man gemäß Verfahren (G_{β}) 2-(2,3,4,6-Tetramethylphenyl)-3-hydroxy-4-trifluormethyl-4-methyl-Δ²-cyclopentenon und Dimethylcarbamidsäurechlorid als Ausgangsprodukte, so kann der Reaktionsverlauf durch folgendes Schema wiedergegeben werden:

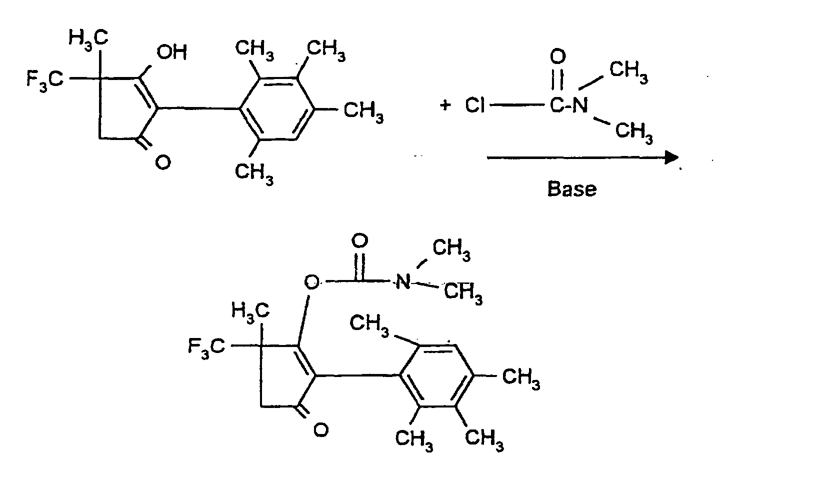

Die bei dem obigen Verfahren (A) als Ausgangsstoffe benötigten Verbindungen der Formel (II) 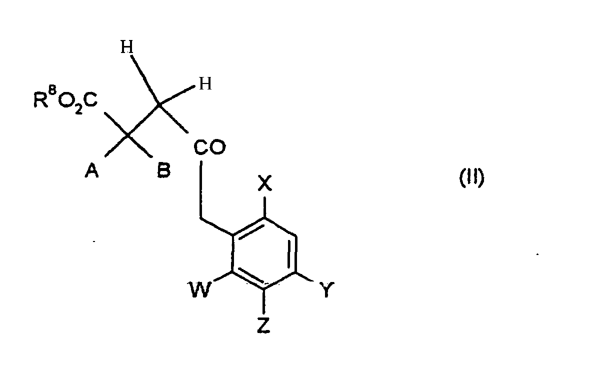 in welcher
A, B, W, X, Y, Z, n und R⁸ die oben angegebene Bedeutung haben,
sind neu.

Sie lassen sich nach im Prinzip bekannten Methoden herstellen.

Man erhält die 5-Aryl-4-ketocarbonsäureester der Formel (II) beispielsweise, wenn man 5-Aryl-4-ketocarbonsäuren der Formel (XIII) 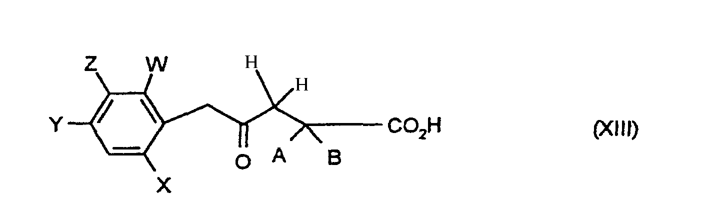 in welcher
W, X, Y, Z, A, und B, die oben angegebene Bedeutung haben,
verestert (vgl. z.B. Organikum, 15. Auflage, Berlin, 1977, Seite 499) oder alkyliert (siehe Herstellungsbeispiel).

Die 5-Aryl-4-ketocarbonsäuren der Formel (XIII) 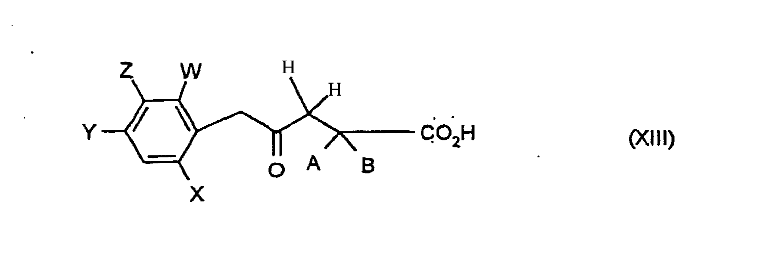 in welcher
A, B, W, X, Y und Z die oben angegebene Bedeutung haben,
sind neu, lassen sich aber nach im Prinzip bekannten Methoden herstellen (siehe Herstellungsbeispiel).

Man erhält die 5-Aryl-4-ketocarbonsäuren der Formel (XIII) beispielsweise, wenn man Carbonsäureanhydride der Formel (XIV) 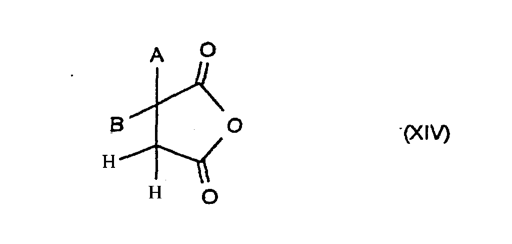 in welcher
- A, und B,: die oben angegebene Bedeutung haben,
mit metallorganischen Verbindungen der Formel (XV) 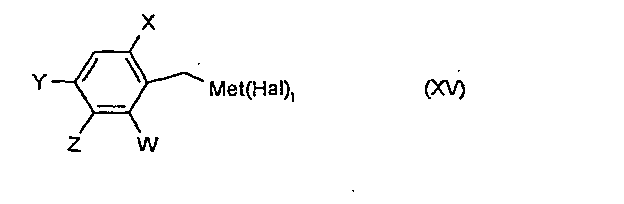 in welcher
- W, X, Y und Z: die oben angegebene Bedeutung haben,
- Met: für ein- oder zweiwertige Metallionen, beispielsweise des Lithiums oder Magnesiums,
- Hal: für Chlor oder Brom
und
- 1: für eine Zahl 0 oder 1 steht,
in Gegenwart eines Verdünnungsmittels umsetzt (vgl. z.B. Organikum, 15. Auflage, Berlin, 1977, Seite 623).

Die Verbindungen der Formeln (XIV) und (XV) sind teilweise bekannt und/oder lassen sich nach bekannten Verfahren darstellen (vgl. z.B. Organikum, 15. Auflage, Berlin, 1977, Seiten 525, 526 und 623).

Weiterhin erhält man 5-Aryl-4-ketocarbonsäuren der Formel (XIII) 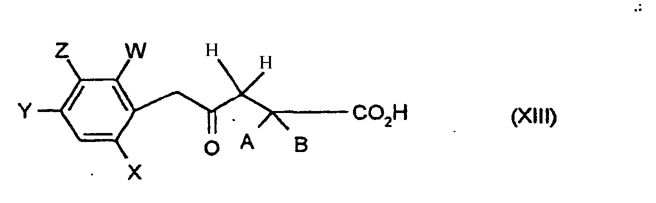 in welcher
A, B, W, X, Y und Z die oben angegebene Bedeutung haben,
wenn man 2-Phenyl-3-oxo-adipinsäureester der Formel (XVI) 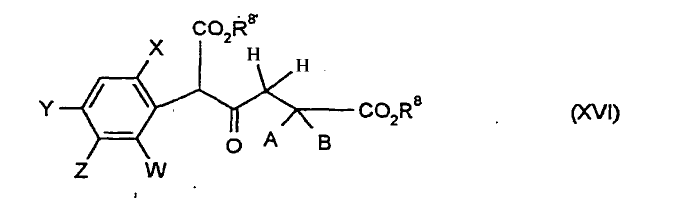 in welcher
A, B, W, X, Y und Z die oben angegebene Bedeutung haben und
- R⁸ und R^{8'}: für Alkyl (insbesondere C₁-C₈-Alkyl) stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XVI) 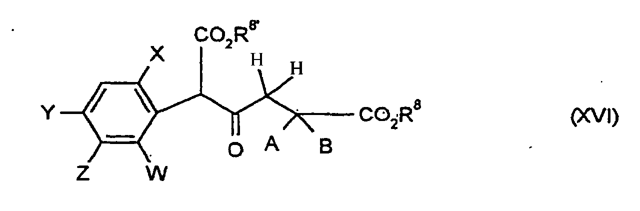 in welcher
A, B, W, X, Y, Z, R⁸, R^{8'} die oben angegebene Bedeutung haben,
sind neu.

Man erhält die Verbindungen der Formel (XVI) beispielsweise,
wenn man Dicarbonsäurehalbesterchloride der Formel (XVII), 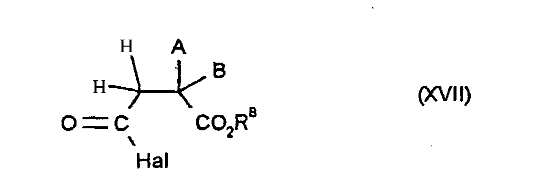 in welcher
A, B, und R⁸ die oben angegebene Bedeutung haben und
- Hal: für Chlor oder Brom steht,
oder Carbonsäureanhydride der Formel (XIV) 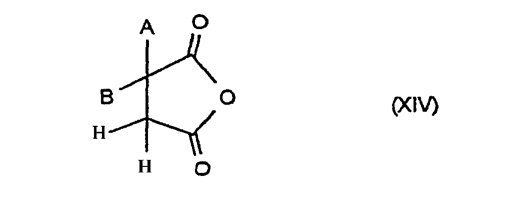 mit einem Phenylessigsäureester der Formel (XVIII) 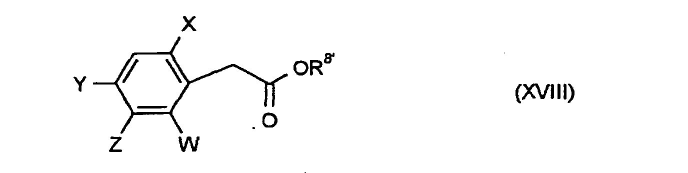 in welcher
- W, X, Y, Z und R^{8'}: die oben angegebene Bedeutung haben,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228, vgl. auch die Herstellungsbeispiele).

Die Verbindungen der Formeln (XVII) sind teilweise bekannte Verbindungen der Organischen Chemie und/oder lassen sich nach im Prinzip bekannten Methoden in einfacher Weise herstellen.

Die Verbindungen der Formel (XVIII) sind teilweise bekannt (WO 97/36868, DE 196 31 586, WO 97/01535, DE 19 02 524) oder lassen sich nach dem dort beschriebenen Verfahren herstellen.

Das Verfahren (A) ist dadurch gekennzeichnet, daß man Verbindungen der Formel (II), in welcher A, B, D¹, D², W, X, Y, Z und R⁸ die oben angegebene Bedeutung haben, in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden. , Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen -75°C und 250°C, vorzugsweise zwischen -50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuß (beispielsweise bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Bβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Bβ) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bβ) werden die Ausgangsstoffe der Formel (I-a) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (I-a) mit Chlorameisensäureestern oder Chlorameisensäurethioestem der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (C) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (C) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (I-a) und der entsprechende Chlorameisensäureester bzw. Chlorameisensäurethioester der Formel (V) im allgemeinen jeweils in angenähert äquivalenten Mengen verwendet. Es ist jedoch auch möglich, die eine oder andere Komponente in einem größeren Überschuß (beispielsweise bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (G) ist dadurch gekennzeichnet, daß man Verbindungen der Formel (I-a) mit (Gα) Verbindungen der Formel (XI) gegebenenfalls in Gegenwart eines Verdünnungsmittels und gegebenenfalls in Gegenwart eines Katalysators oder (Gα) mit Verbindungen der Formel (XII) gegebenenfalls in Gegenwart eines Verdünnungsmittels und gegebenenfalls in Gegenwart eines Säurebindemittels umsetzt.

Bei Herstellungsverfahren (Gα) setzt man pro Mol Ausgangsverbindung der Formel (I-a) ca. 1 Mol Isocyanat der Formel (XI) bei 0 bis 100°C, vorzugsweise bei 20 bis 50°C um.

Das Verfahren (Gα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Gβ) setzt man pro Mol Ausgangsverbindung der Formel (I-a) ca. 1 Mol Carbamidsäurechlorid der Formel (XII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantü, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) oder gegen die Raupen der Kohlschabe (Plutella maculipennis) einsetzen.

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfaibstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-(2-[6-(2-cyanophenoxy)-pyrimidin-4-yloxy]-phenyl}-3-methoxyacrylat; Methyl-(E)-methoximino[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Brömuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazal,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primiselfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausfuhrungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole; Cyproconazole, Metconazole, Imazalil, Dichlofluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den folgenden Beispielen hervor.

### Herstellungsbeispiele

### Beispiel Ia-1:

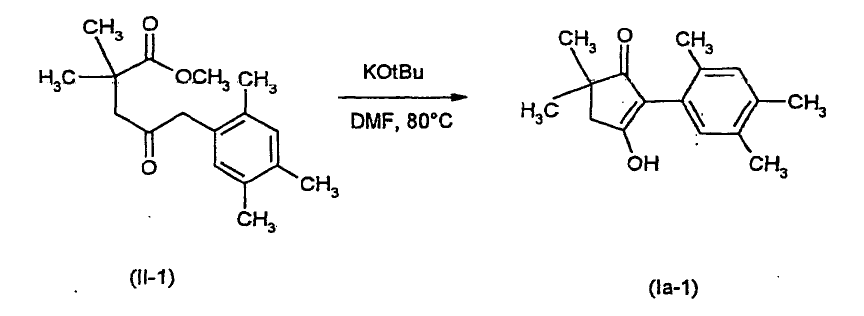

Zu einer Lösung der Verbindung gemäß Beispiel II-1 (4.4 g, 16 mmol) in DMF (30 ml) gibt man 2.7 g (24 mmol) Kaliumtertbutylat und rührt 1 Stunde bei 80°C. Man rührt in ca. 600 ml 1 NHCl unter Eisbadkühlung ein, saugt den Feststoff ab und trocknet.
Rohausbeute: 4 g (=̂ 100 % d. Theorie). Fp. 185-186°C.

Analog bzw. gemäß den allgemeinen Angaben zur Herstellung wurden die in der Tabelle 27 aufgeführten Verbindungen der Formel (Ia) hergestellt, die in Form eines der möglichen Isomeren dargestellt sind.

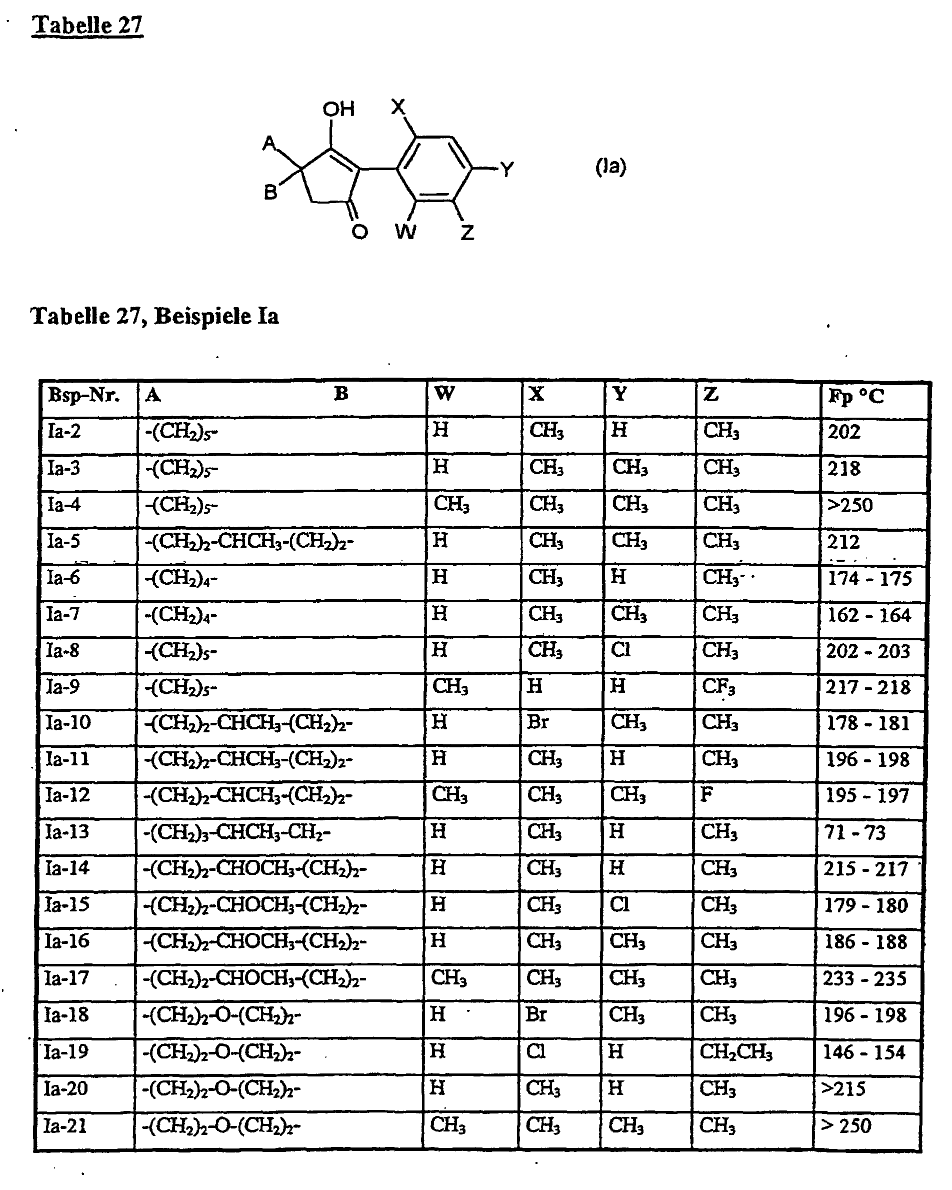

### Beispiel Ib-1:

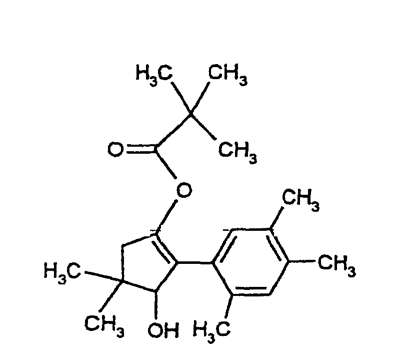

1,5 g (6,0 mmol) der Verbindung gemäß Beispiel Ia-1 werden in 20 ml trockenem Methylenchlorid vorgelegt und mit 1,25 ml (9,0 mmol; 1,5 eq) Triethylamin versetzt.

Eine Lösung von 1,16 ml (1,14 g, 9,0 mmol; 1,5 eq) Pivaloylchlorid in 3 ml trockenem Methylenchlorid wird unter Eiskühlung zugetropft. Es wird 1 bis 2 Stunden bei Raumtemperatur gerührt, 2 mal mit 10 %iger Citronensäure gewaschen und die vereinigten wäßrigen sauren Phasen mit Methylenchlorid extrahiert. Die vereinigten organischen Phasen werden 2 mal mit 1 N NaOH gewaschen und die wäßrigen alkalischen Phasen mit Methylenchlorid extrahiert. Die vereinigten organischen Phasen werden getrocknet und eingeengt.

Ausbeute: 1,90 g (96,00 % d. Theorie). Fp. 75-77°C.

Analog bzw. gemäß den allgemeinen Angaben zur Herstellung wurden die in der Tabelle 28 aufgeführten Verbindungen der Formel (Ib) hergestellt, die in Form eines der möglichen Isomeren dargestellt sind.

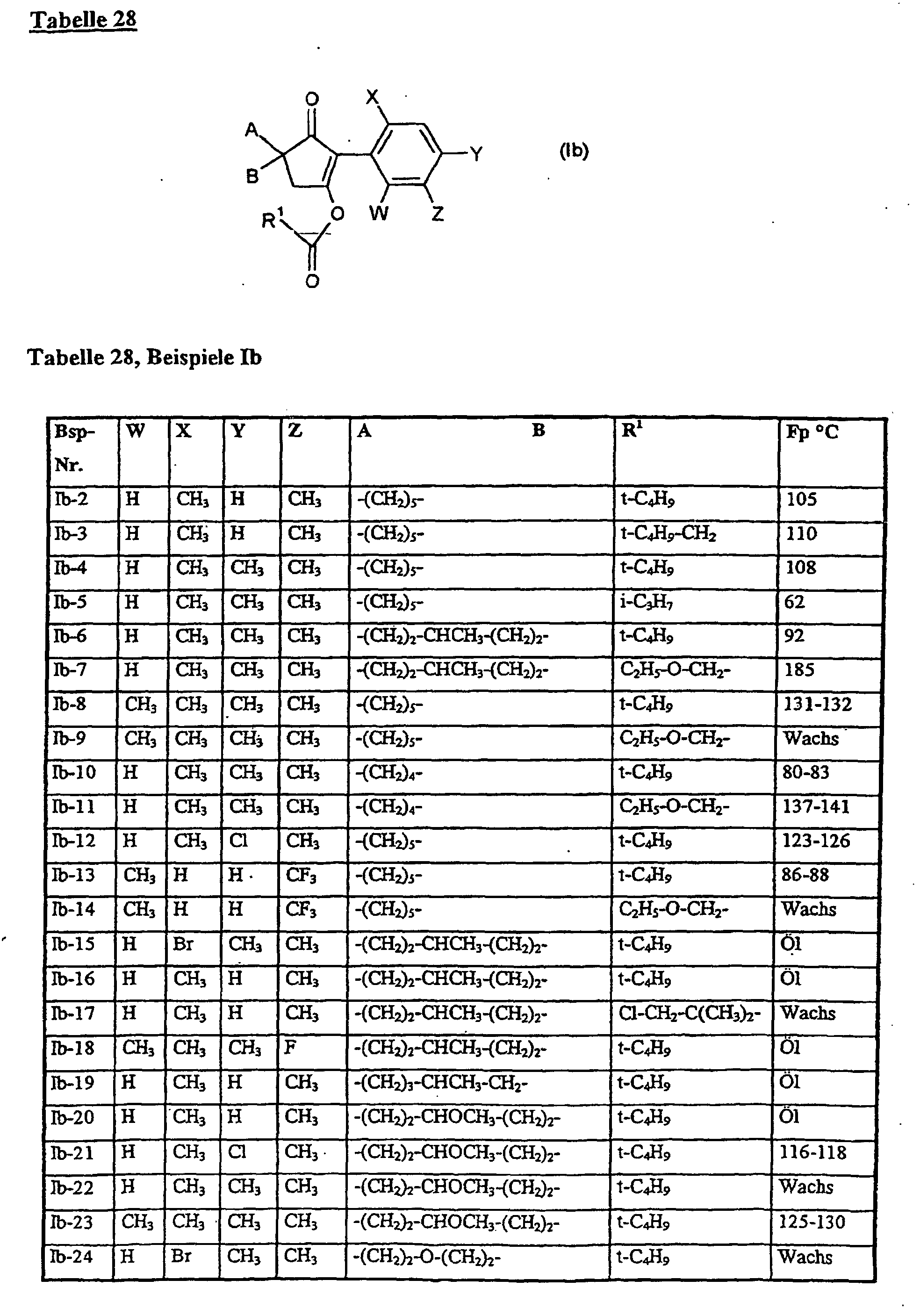

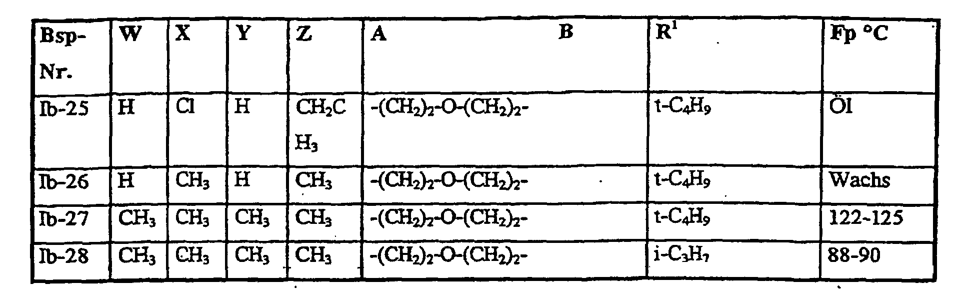

### Beispiel Ic-1:

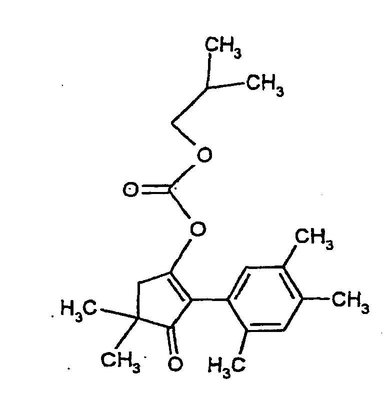

1,5 g (6,0 mmol) der Verbindung gemäß Beispiel Ia-1 werden in 20 ml trockenem Methylenchlorid vorgelegt und mit 1,25 ml (9,0 mmol; 1,5 eq) Triethylamin versetzt.

Eine Lösung von 1,17 ml (1,23 g, 9,0 mmol; 1,5 eq) Chlorameisensäureisobutylester in 3 ml trockenem Methylenchlorid wird unter Eiskühlung zugetropft. Es wird 1 bis 2 Stunden bei Raumtemperatur gerührt.

Es wird wie bei Beispiel Ib-1 beschrieben aufgearbeitet.
Ausbeute: 2,00 g Öl. 97 % der Theorie.
¹H-NMR (400 MHz, CDCl₃): 0,9 (d, 6H, CH(CH₃)₂); 1,2 (s, 6H, CH(CH₃)₂); 1,9 (m, 1H, CH(CH₃)₂); 2,1 (s, 3H, ArCH₃); 2,2 (d, 6H, 2 x ArCH₃), 2,9 (s, 2H, CH₂); 3,9 (d, 2H, OCH₂); 6,8; 7,0 (s, 2H, Ar-H).

Analog bzw. gemäß den allgemeinen Angaben zur Herstellung wurden die in der Tabelle 29 aufgeführten Verbindungen der Formel (Ic) hergestellt, die in Form eines der möglichen Isomeren dargestellt sind.

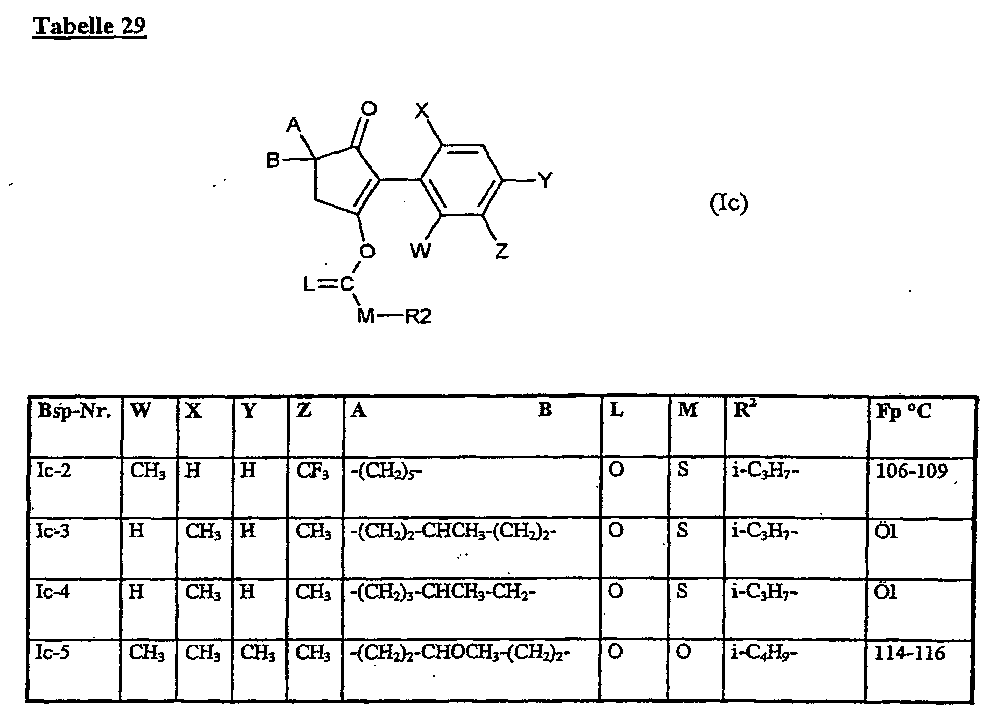

### Beispiel If-1

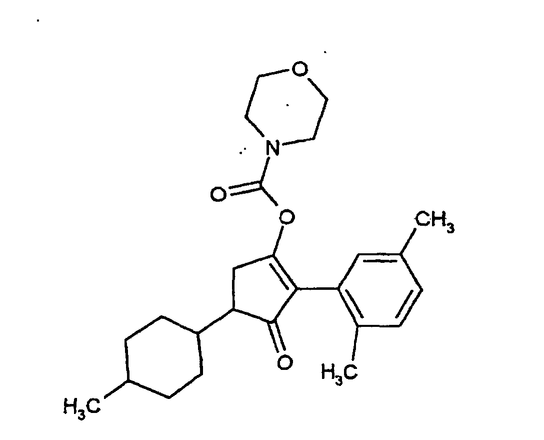

1,0 g (3,5 mmol) der Verbindung gemäß Beispiel Ia-11 werden in 30 ml trockenem Ethylacetat vorgelegt und mit 0,73 ml (5,2 mmol; 1,5 eq) Triethylamin versetzt.

Eine Lösung von 0,53 ml (0,68 g; 4,6 mmol; 1,3 eq) Morpholin-N-carbonsäurechlorid in 5 ml trockenem Ethylacetat wird bei Raumtemperatur zugetropft. Es wird 4 Stunden unter Rückfluß erhitzt.

Der Ansatz wird am Rotationsverdampfer eingeengt und in Methylenchlorid aufgenommen. Es wird 2 mal mit 0,5 N NaOH gewaschen. Die organische Phase wird getrocknet und eingeengt.
Ausbeute: 1,0 g (72 % d. Theorie) Öl.
¹H-NMR (400 MHz, DMSO-d₆): 0,95 (d, 3H, CHCH₃); 1,3 - 1,8 (m, 9H, Cyclohexyl-H); 2,05 (s, 3H, ArCH₃); 2,25 (s, 3H, ArCH₃); 2,9 (s, 2H, CH₂); 3,2 - 3,8 (m, 8H, Morpholin-H); 6,85 (s, 1H, Ar-H), 7,05 (d, 1H, Ar-H); 7,12 (d, 1H, Ar-H).

### Beispiel (II-1):

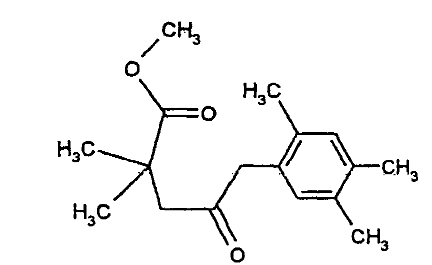

Zu 12,2 g (46,5 mmol) der Verbindung gemäß Beispiel (XIII-1) (Rohprodukt) in 100 ml absolutem Aceton gibt man 6,40 g (46,5 mmol) Kaliumcarbonat und 19,78 g (139,5 mmol) Methyliodid und rührt 16 Stunden unter Rückfluß. Man filtriert und engt im Vakuum ein. Der Rückstand (11,9 g) wird an Kieselgel und Methylenchlorid/Petrolether 8/1 bis 1/0 chromatographiert.
Ausbeute: 4,70 g (47 % der Theorie), Öl.
¹H-NMR (400 MHz, CDCl₃): 1,1 (s, 6H, C(CH₃)₂); 2,1 (s, 3H, ArCH₃); 2,2 (s, 6H, 2×ArCH₃); 2,7 (s, 2H, CH(CMe₂); 3,5(s, 2H, ArCH₂), 3,6 (s, 3H, OCH₃); 6,8 / 6,9 (s, 2H, 2xArCH).

Analog zu Beispiel (II-1) bzw. gemäß den allgemeinen Angaben zur Herstellung wurden die in der Tabelle 30 aufgeführten Verbindungen der Formel (II) hergestellt.

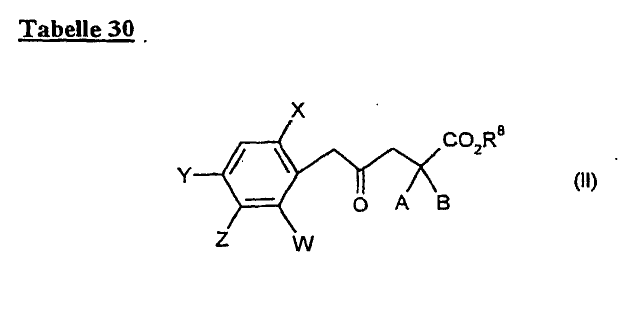

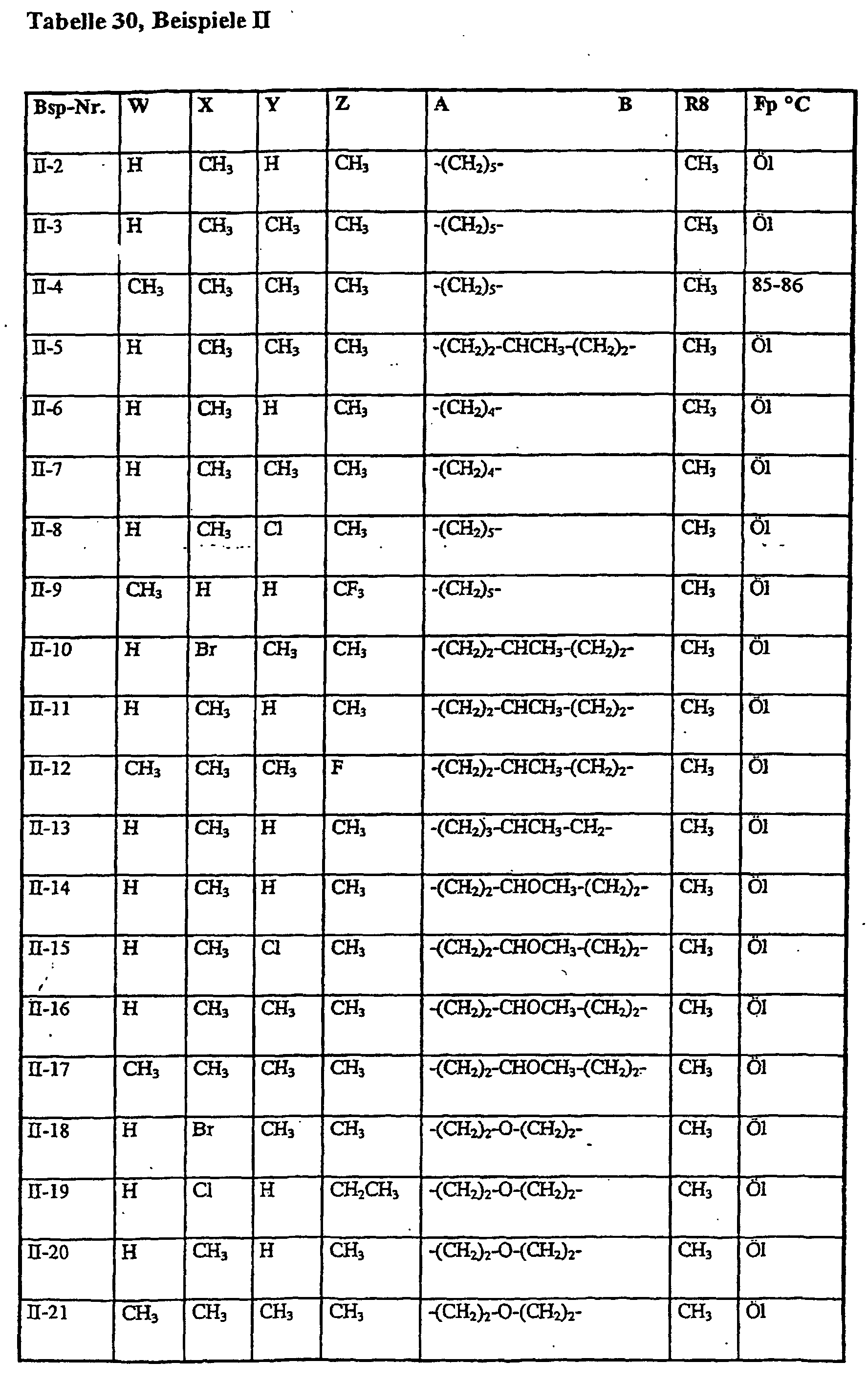

### Beispiel XIII-1

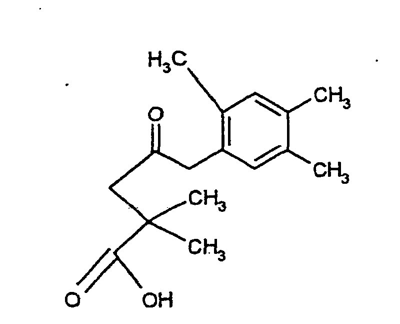

Zu einer Lösung von 30,0 ml LDA (Lithiumdiisopropylamid)-Lösung (2 molar; 1,1 eq) in 60 ml Tetrahydrofuran (THF) p.a. wird eine Lösung von 10,3 g (54 mmol; 1 eq) 2,4,5-Trimethyl-phenylessigsäure-methylester in 20 ml THF p.a. bei -15°C zugetropft und 30 min bei dieser Temperatur gerührt.

Dann wird bei -15°C eine Lösung von 4,62 g (36 mmol; 0,66 eq) 2,2-Dimethylbernsteinsäureanhydrid in 20 ml THF p.a. zugetropft.

Es wird zwei Stunden bei Raumtemperatur gerührt, dann werden 75 ml Wasser und 20 g Ammoniumchlorid zugegeben.

Das Zwischenprodukt wird mit Ether extrahiert, die Lösungsmittel im Vakuum entfernt. Der Rückstand wird mit 50 g KOH und 165 ml Wasser zwei Tage unter Rückfluß gekocht.

Man kühlt ab, säuert mit konzentrierter HCl an und extrahiert mit Ether. Das Rohprodukt wird direkt weiter umgesetzt.
Ausbeute: 12,20 g, 100,00 % d. Theorie. Fp. 112-115°C.

Analog zu Beispiel XIII-I bzw. gemäß den allgemeinen Angaben zur Herstellung wurden die in der Tabelle 31 aufgeführten Verbindungen der Formel (XIII) hergestellt.

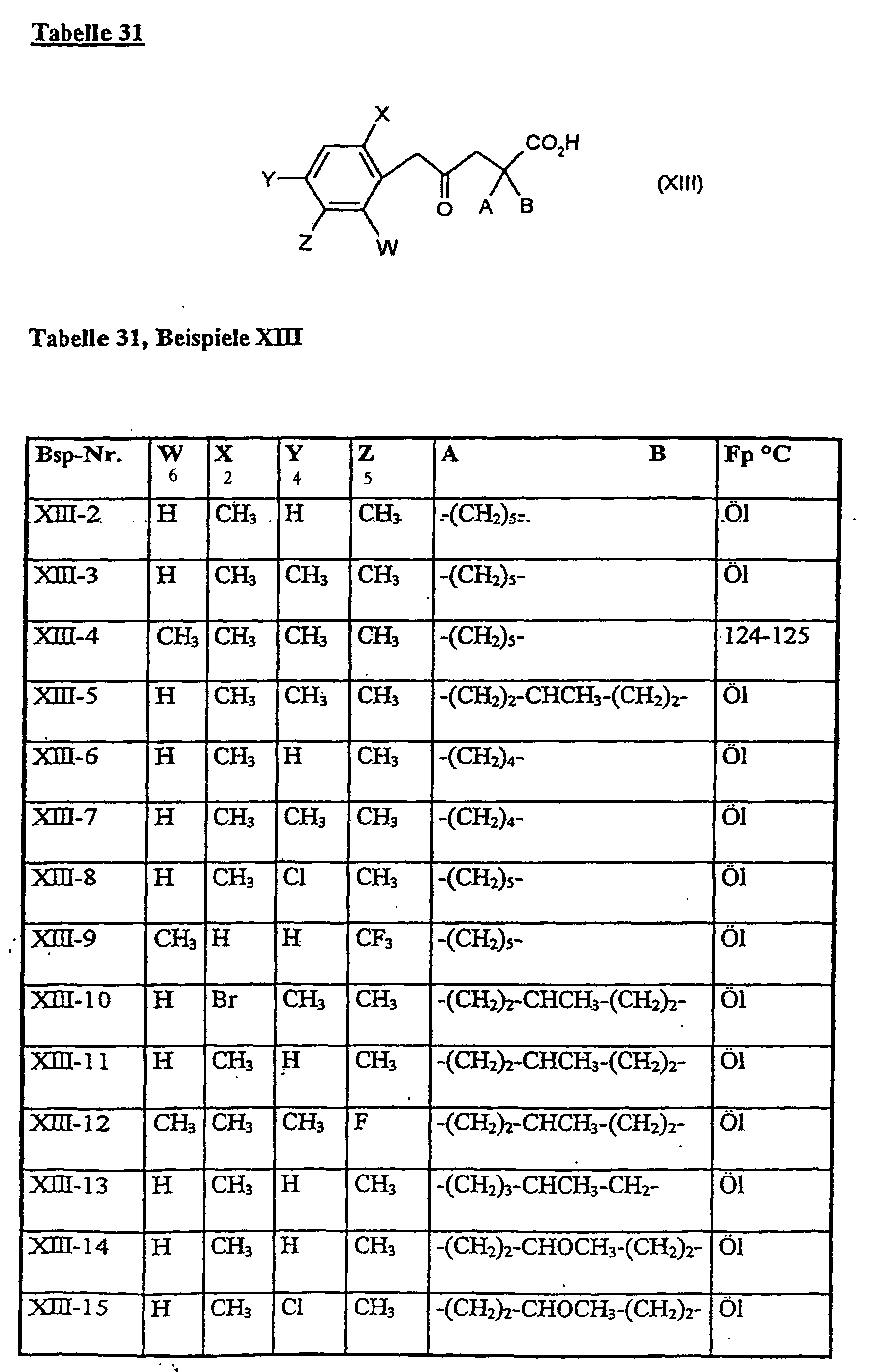

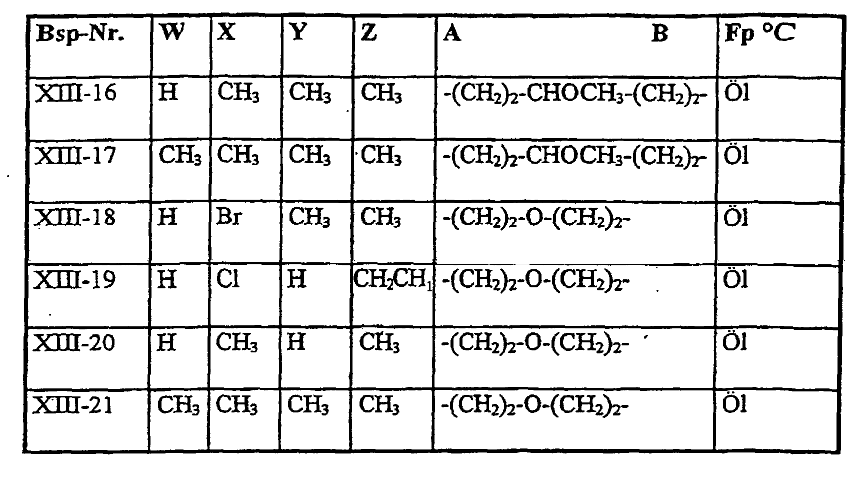

### Beispiel (XIV-1):

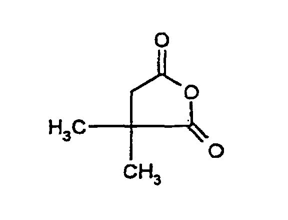

50,0 g (0,342 mol) 2,2-Dimethylbernsteinsäure werden in 250 ml Essigsäureanhydrid über Nacht unter Rückfluß gekocht:

Es wird filtriert, am Rotationsverdampfer eingeengt und 2 mal mit Toluol kodestilliert. Der Rückstand wird in wenig Methylenchlorid gelöst, mit n-Hexan versetzt, über Nacht im Eiskühlschrank aufbewahrt, abgesaugt und getrocknet.
Ausbeute: 36,40 g (83,00 % d. Theorie), Öl.
¹H-NMR (CDCl₃, 500 Mhz): 1,5 (s, 6H, CH₃), 2,9 (s, 2H, CH₂).

### Beispiel 1

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit der Grünen Reiszikade (Nephotettix cincticeps) besetzt, solange die Blätter noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß den Herstellungsbeispielen Ia-2, Ia-3, Ia-4, Ia-5, Ib-1, Ib-2, Ib-3, Ib-4, Ib-5, Ib-8, Ib-9 und Ic-1 einer beispielhaften Wirkstoffkonzentration von 0,1 % eine Abtötung von 100 % nach 6 Tagen.

### Beispiel 2

### Phaedon-Larven-Test

| | |
|---|---|
| Lösungsmittel | 7 Gewichtsteile Dimethylformamid |
| Emulgator | 1 Gewichtsteil Alkylarylpolyglykolether, |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Larven des Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test bewirkten z.B. die Verbindungen gemäß den Herstellungsbeispielen Ia-1, Ib-2, Ib-4, Ib-5 und Ib-9 bei einer beispielhaften Wirkstoffkonzentration von 0, 1 % ein Abtötung von 100 % nach 7 Tagen.

### Beispiel 3

### Tetranychus-Test (OP-resistent/Tauchbehandlung)

| | |
|---|---|
| Lösungsmittel | 7 Gewichtsteile Dimethylformamid. |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Stadien der gemeinen Spinnmilbe Tetranychus urticae befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test hatten z.B. die Verbindungen der Herstellungsbeispiele Ia-2, Ia-3, Ib-1, Ib-2, Ib-3, Ib-4, Ib-5, Ib-8 und Ic-1 bei einer beispielhaften Wirkstoffkonzentration von 0,01 % eine Wirkung von 100 % nach 7 Tagen.

### Beispiel 4

### Test mit Boophilus microplus resistent/SP-resistenter Parkhurst-Stamm

| | |
|---|---|
| Testtiere | adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen in dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test hatten z.B. die Verbindungen gemäß den Herstellungsbeispielen Ia-2 und Ia-3 bei einer beispielhaften Wirkstoffkonzentration von 20 µg/Tier eine Wirkung von jeweils 100 %.

## Patentansprüche

1. Verbindungen der Formel (I): 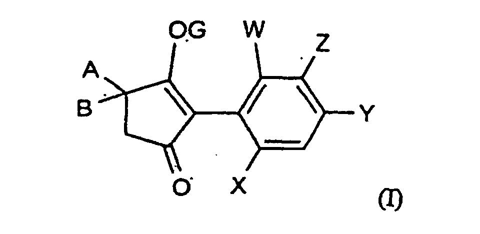 in welcher die Substituenten die nachstehend angegebenen Bedeutungen haben:
W = CH₃; X = CH₃; Y = CH₃; Z = CH₃ oder
W = CH₃; X = CH₃; Y = H; Z = Cl oder.
W = CH₃; X = CH₃; Y = CH₃; Z = F oder
W = CH₃; X = CH₃; Y = CH₃; Z = Cl oder
W = CH₃; X = CH₃; Y = CH₃; Z = Br oder.
W = CH₃; X = CH₃; Y = H; Z = Broder
W = Cl; X = Cl; Y = H; Z = Br oder
W = Br; X = Br; Y, Z = -(CH₂)₃- oder.
W = CH₃; X = OCH₃; Y = H; Z = Br oder
W = CH₃; X = CH₃; Y = CH₃; Z = CH₃ oder
W = Cl; X = Cl; Y = Cl; Z = CH₃ oder
W = Br; X = Br; Y = Br; Z = CH₃ oder
W = H; X = Cl; Y = Cl; Z = Cl oder
W = H; X = CH₃; Y = CH₃, Z = CH₃ oder
W = H, X = CH₃; Y = Cl, Z = CH₃ oder
W = H; X = Br; Y = CH₃; Z = Cl oder
W = H; X = Br; Y = CH₂ ; Z = CH₂ oder
W = H; X = Cl; Y = Br, Z = CH₃ oder
W = H; X = Cl, Y = Cl; Z = CH₃ oder
W = H; X = CH₃; Y = CH₃; Z = Br oder
W = H; X = Cl; Y, Z = -O-CF₂-O- oder
W = H; X = Br; Y = CH₃; Z = Br oder
W=H; X=CH₃; Y=H; 2=CH₃ oder
W = H; X = Cl; Y = H; Z = NO₂ oder
W = H; X = Br; Y = H; Z = OCH₃,
G = H,
| **A** | **B** |
|---|---|
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -(CH₂)₂-S-(CH₂)₂- | |
| -CH₂-O-(CH₂)₃- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| - (CH₂)₂-CHOC₃H₇-(CH₂)₂- | |
| - (CH₂)₂-CHOi-C₃H₇-(CH₂)₂- | |
| -(CH₂)₂-C(CH₃)₂-(CH₂)₂- | |
| -CH₂-(CHCH₃)₂-(CH₂)₂- | |

2. Verbindungen der Formel (I) bitte Formel (I) einfügen, in welcher die Substituenten die in der Tabelle angegebenen Bedeutungen haben

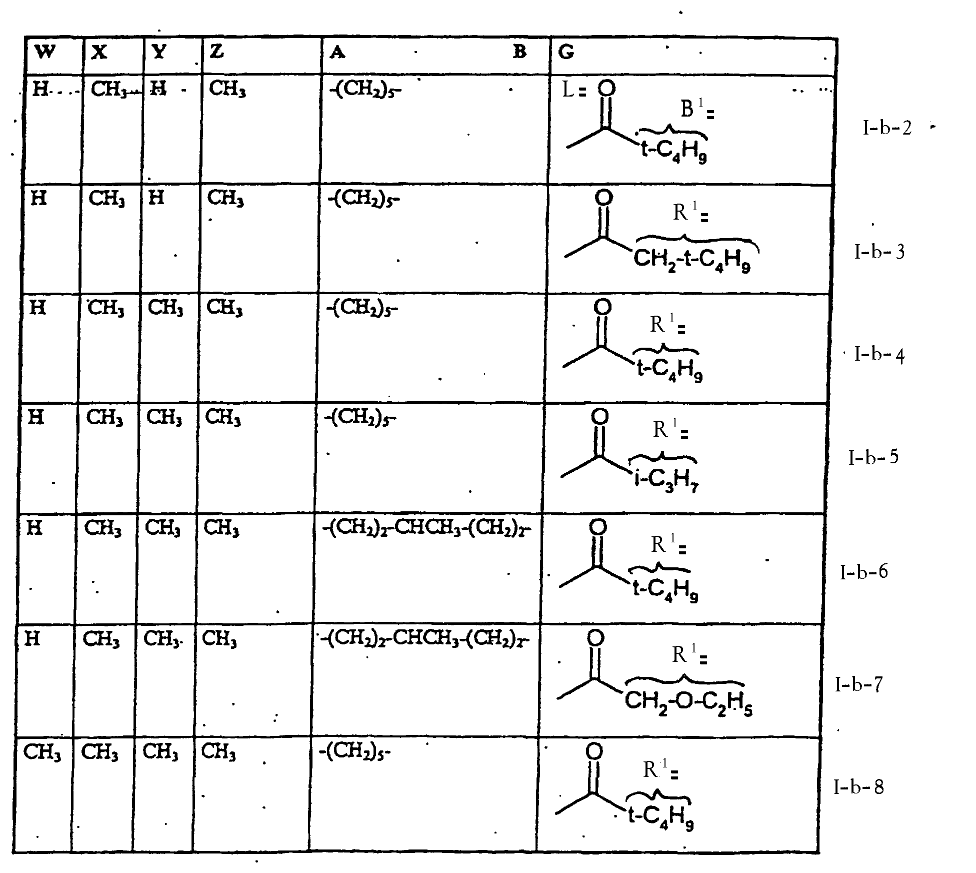

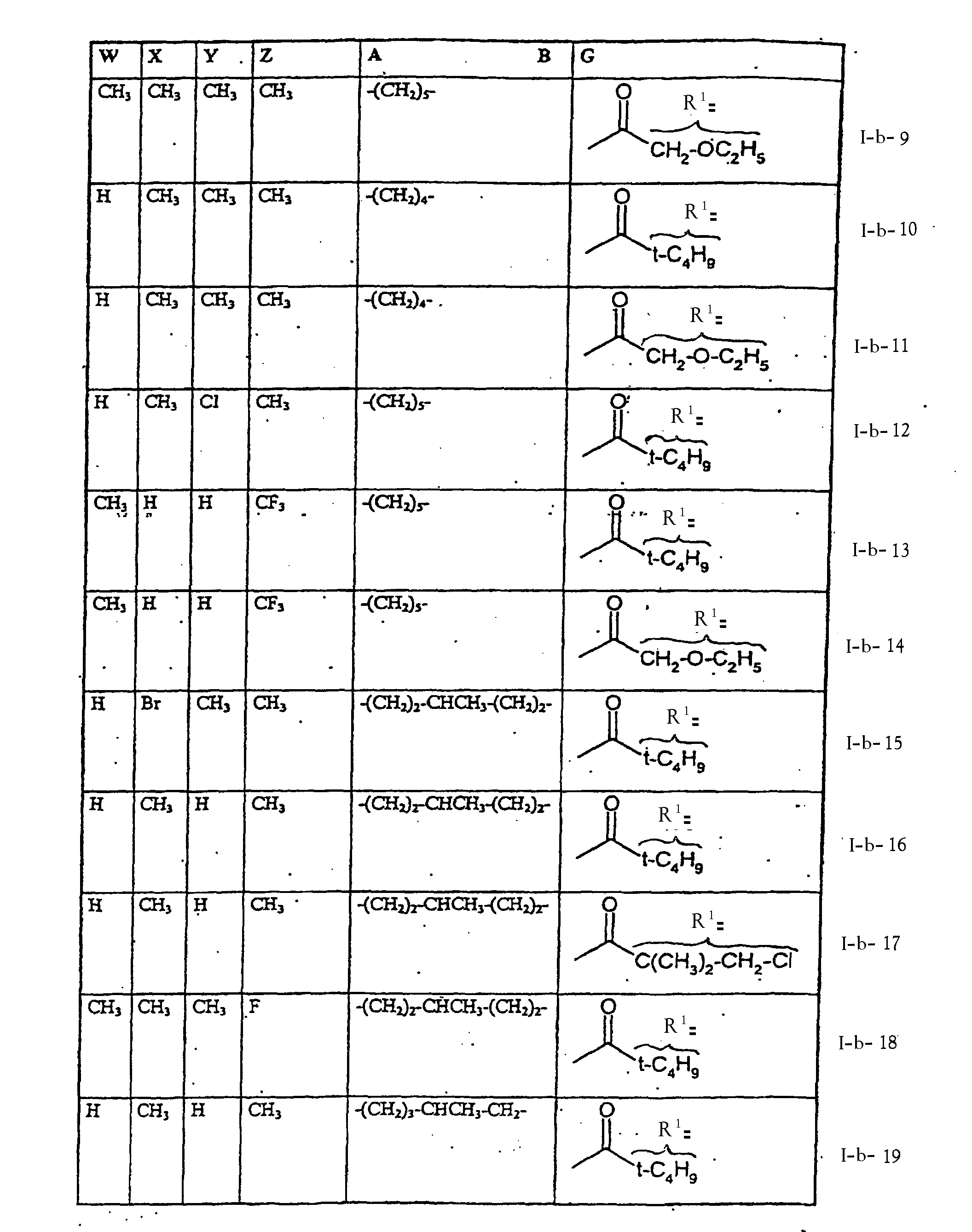

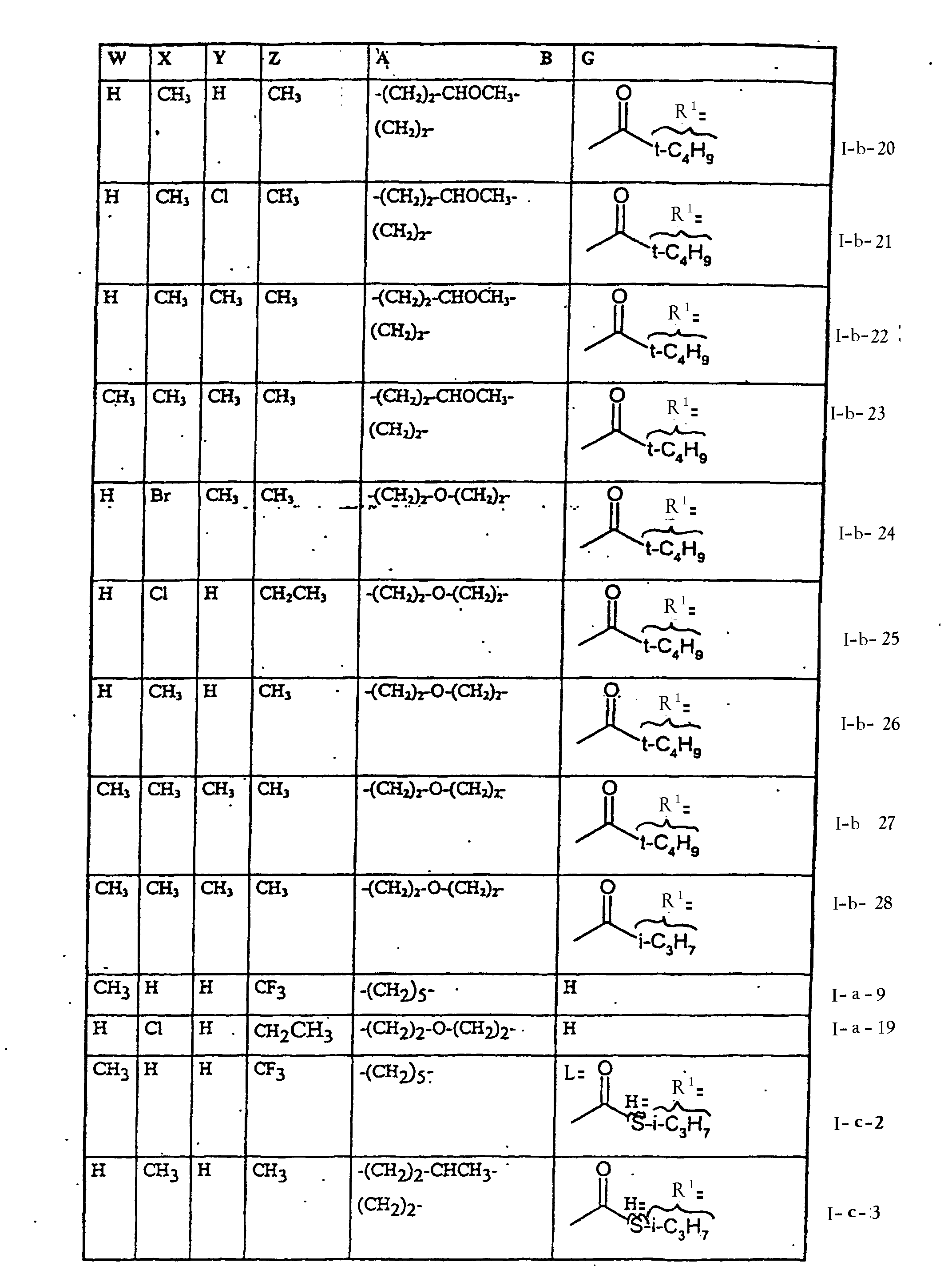

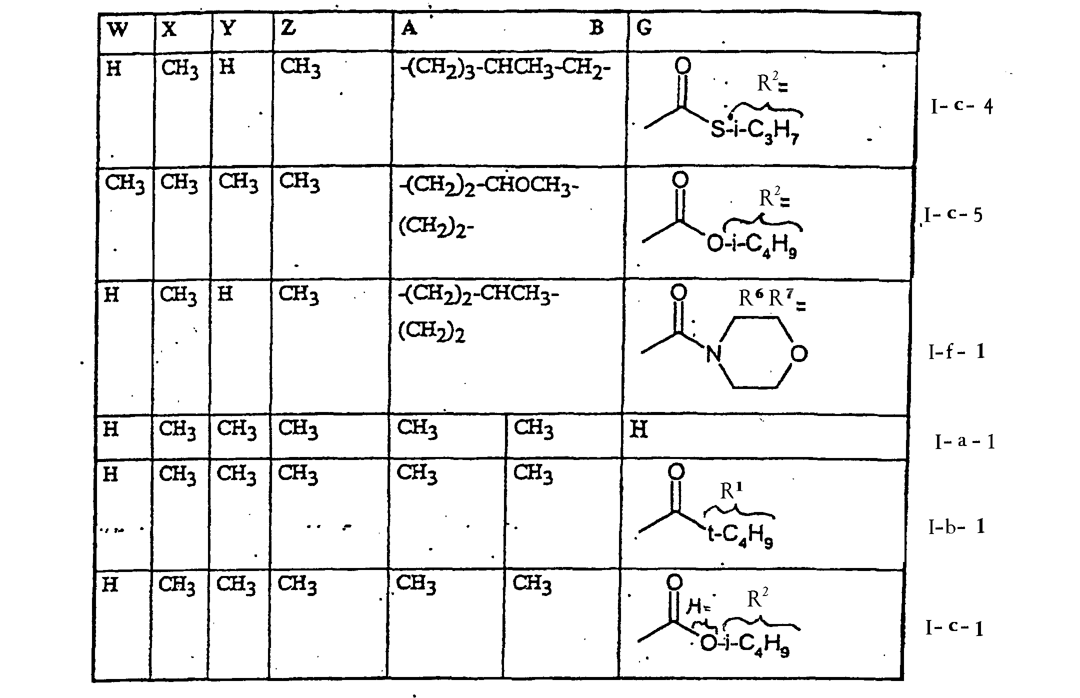

3. Verbindungen der Formel (II) 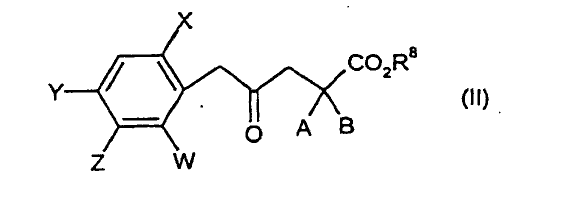 in welcher die Substituenten die in der Tabelle angegebenen Bedeutungen haben
| **W** | **X** | **Y** | **Z** | **A** | **B** | **R8** | |
|---|---|---|---|---|---|---|---|
| H | CH₃ | H | CH₃ | -(CH₂)₅- | | CH₃ | II-2 |
| H | CH₃ | CH₃ | CH₃ | -(CH₂)₅- | | CH₃ | II-3 |
| CH₃ | CH₃ | CH₃ | CH₃ | -(CH₂)₅- | | CH₃ | II-4 |
| H | CH₃ | CH₃ | CH₃ | -(CH₂)₂-CHCH₃-(CH₂)₂ | | CH₃ | II-5 |
| H | CH₃ | H | CH₃ | -(CH₂)₄- | | CH₃ | II-6 |
| H | CH₃ | CH₃ | CH₃ | -(CH₂)₄- | | CH₃ | II-7 |
| H | CH₃ | Cl | CH₃ | -(CH₂)₅- | | CH₃ | II-8 |
| CH₃ | H | H | CF₃ | -(CH₂)₅- | | CH₃ | II-9 |
| H | Br | CH₃ | CH₃ | -(CH₂)₂-CHCH₃-(CH₂)₂ | | CH₃ | II-10 |
| H | CH₃ | H | CH₃ | -(CH₂)₂-CHCH₃-(CH₂)₂- | | CH₃ | II-11 |
| CH₃ | CH₃ | CH₃ | F | -(CH₂)₂CHCH₃-(CH₂)₂- | | CH₃ | II-12 |
| H | CH₃ | H | CH₃ | -(CH₂)₃-CHCH₃-CH₂- | | CH₃ | II-13 |
| H | CH₃ | H | CH₃ | -(CH₂)₂-CHOCH₃-(CH₂)₂- | | CH₃ | II-14 |
| H | CH₃ | Cl | CH₃ | -(CH₂)₂-CHOCH₃-(CH₂)₂- | | CH₃ | II-15 |
| H | CH₃ | CH₃ | CH₃ | -(CH₂)₂-CHOCH₃-(CH₂)₂- | | CH₃ | II-16 |
| CH₃ | CH₃ | CH₃ | CH₃ | -(CH₂)₂-CHCCH₃-(CH₂)₂- | | CH₃ | II-17 |
| H | Br | CH₃ | CH₃ | -(CH₂)₂-O-(CH₂)₂- | | CH₃ | II-18 |
| H | Cl | H | CH₂CH₃ | -(CH₂)₂-O-(CH₂)₂- | | CH₃ | II-19 |
| H | CH₃ | H | CH₃ | -(CH₂)₂-O-(CH₂)₂- | | CH₃ | II-20 |
| CH₃ | CH₃ | CH₃ | CH₃ | -(CH₂)₂-O-(CH₂)₂- | | CH₃ | II-21 |
| H | CH₃ | CH₃ | CH₃ | CH₃ | CH₃ | CH₃ | II-1 |

4. Verbindungen der Formel (XIII) 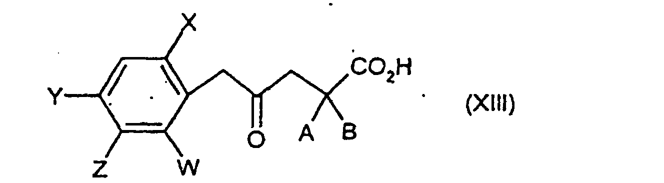 in welcher die Substituenten die in der Tabelle angegebenen Definitionen haben
| **W** | **X** | **Y** | **Z** | **A** | **B** | |
|---|---|---|---|---|---|---|
| H | CH₃ | H | CH₃ | -(CH₂)₅- | | XIII-2 |
| H | CH₃ | CH₃ | CH₃ | -(CH₂)₅- | | XIII-3 |
| CH₃ | CH₃ | CH₃ | CH₃ | -(CH₂)₅- | | XIII-4 |
| H | CH₃ | CH₃ | CH₃ | -(CH₂)₂-CHCH₃-(CH₂)₂- | | XIII-5 |
| H | CH₃ | H | CH₃ | -(CH₂)₄- | | XIII-6 |
| H | CH₃ | CH₃ | CH₃ | -(CH₂)₄- | | XIII-7 |
| H | CH₃ | Cl | CH₃ | -(CH₂)₅- | | XIII-8 |
| CH₃ | H | H | CF₃ | -(CH₂)₅- | | XIII-9 |
| H | Br | CH₃ | CH₃ | -(CH₂)₂-CHCH₃-(CH₂)₂- | | XIII-10 |
| H | CH₃ | H | CH₃ | -(CH₂)₂-CHCH₃-(CH₂)₂- | | XIII-11 |
| CH₃ | CH₃ | CH₃ | F | -(CH₂)₂CHCH₃-(CH₂)₂- | | XIII-12 |
| H | CH₃ | H | CH₃ | -(CH₂)₃-CHCH₃-CH₂- | | XIII-13 |
| H | CH₃ | H | CH₃ | -(CH₂)₂-CHOCH₃-(CH₂)₂- | | XIII-14 |
| H | CH₃ | Cl | CH₃ | -(CH₂)₂-CHOCH₃-(CH₂)₂- | | XIII-15 |
| H | CH₃ | CH₃ | CH₃ | -(CH₂)₂-CHOCH₃-(CH₂)₂- | | XIII-16 |
| CH₃ | CH₃ | CH₃ | CH₃ | -(CH₂)₂-CHCCH₃-(CH₂)₂- | | XIII-17 |
| H | Br | CH₃ | CH₃ | -(CH₂)₂-O-(CH₂)₂- | | XIII-18 |
| H | Cl | H | CH₂CH₃ | -(CH₂)₂-O-(CH₂)₂- | | XIII-19 |
| H | CH₃ | H | CH₃ | -(CH₂)₂-O-(CH₂)₂- | | XIII-20 |
| CH₃ | CH₃ | CH₃ | CH₃ | -(CH₂)₂-O-(CH₂)₂- | | XIII-21 |
| H | CH₃ | CH₃ | CH₃ | CH₃ | CH₃ | XIII-1 |

5. Schädlingsbekämpfungsmittel oder herbizide Mittel, **gekennzeichnet durch** einen Gehalt an einer Verbindung der Formel (I) gemäß Anspruch 1 und 2.

6. Verwendung von Verbindungen der Formel (I) gemäß Anspruch 1 und 2 zur Bekämpfung von Schädlingen und Unkräutern.

7. Verfahren zur Bekämpfung von Schädlingen und Unkräutern, **dadurch gekennzeichnet, daß** man Verbindungen der Formel (I) gemäß Anspruch 1 und 2 auf Schädlinge bzw. Unkräuter und/oder ihren Lebensraum einwirken läßt.

8. Verfahren zur Herstellung von Schädlingsbekämpfungsmitteln und herbiziden Mitteln, **dadurch gekennzeichnet, daß** man Verbindungen der Formel (I) gemäß Anspruch 1 und 2 mit Streckmitteln und/oder oberflächenaktiven Mitteln vermischt.

9. Verfahren zur Herstellung von Verbindungen der Formel (I) gemäß Anspruch 1 und 2, **dadurch gekennzeichnet, daß** man
(A) Verbindungen der Formel (II) 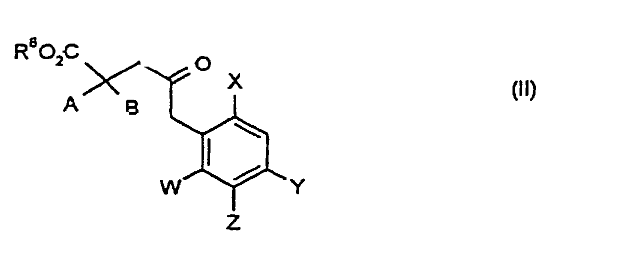 in welcher
A, B, W, X, Y und Z die oben angegebene Bedeutung haben, und
R⁸ für Alkylsteht,
gegebenenfalls in Gegenwart eines Verdünnungsmittels und in Gegenwart einer Base intramolekular cyclisiert und gegebenenfalls anschließend die so erhaltenen Verbindungen der Formel (Ia) 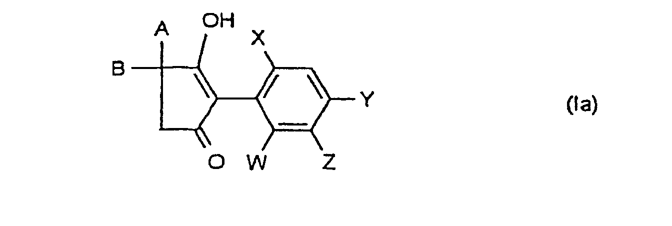 in welcher
A, B, W, X, Y und Z die in Anspruch 1 angegebene Bedeutung haben,
(B)
α) mit Säurehalogeniden der Formel (III) 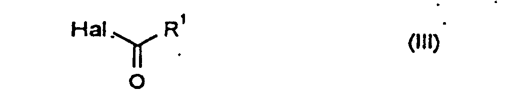 in welcher
R¹ für t-C₄H₉, CH₂-t-C₄H₉ i-C₃H₇, CH₂OC₂H₅ oder C(CH₃)₂CH₂Cl steht und
Hal für Halogen steht,
oder
β) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
oder
(C) mit Chlorameisensäureestern oder Chlorameisensäurethioestern der Formel (V)
R²-M-CO-Cl (V)
in welcher
R² für i-C₃H₇ oder i-C₄H₉ steht und
M für Saüestoff oder Schwefel steht,
gegebenenfalls in Gegenwart eines Verdünnungsmittels und gegebenenfalls in Gegenwart eines Säurebindemittels umsetzt;
oder
(G)
β) mit Carbamidsäurechloriden öder Thiocarbamidsäurechloriden der Formel (XII) 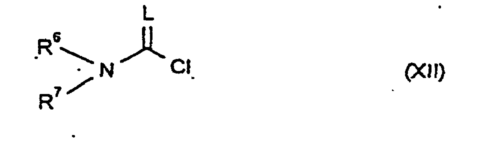 in welcher
L, für Sauerstoff steht und
R⁶ und R⁷ gemeinsam mid dem N-Atom an das sie gebunden sind die Gruppe 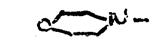 bilden, gegebenenfalls, in Gegenwart eines Verdünnungsmittels und gegebenenfalls in Gegenwart eines Säurebindemittels, umsetzt.

## Claims

1. Compounds of the formula (I): 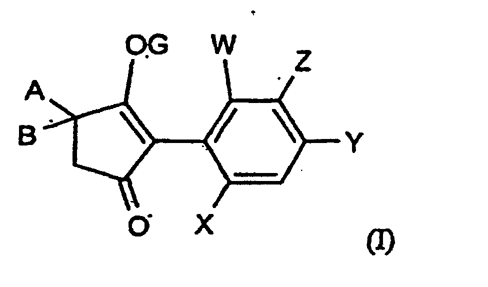 in which the substituents are as defined below:
W = CH₃; X = CH₃; Y = CH₃; Z = CH₃ or
W = CH₃; X = CH₃; Y = H; Z = Cl or
W = CH₃; X = CH₃; Y = CH₃; Z = F or
W = CH₃; X = CH₃; Y = CH₃; Z = Cl or
W = CH₃; X = CH₃; Y = CH₃; Z = Br or
W = CH₃; X = CH₃; Y = H; Z = Br or
W = Cl; X = Cl; Y = H; Z = Br or
W = Br; X = Br; Y, Z = - (CH₂)₃- or
W = CH₃; X = OCH₃; Y = H; Z = Br or
W = CH₃; X = CH₃; Y = CH₃; Z = CH₃ or
W = Cl; X = Cl; Y = Cl; Z = CH₃ or
W = Br; X = Br; Y = Br; Z = CH₃ or
W = H; X = Cl; Y = Cl; Z = Cl or
W = H; X = CH₃; Y = CH₃, Z = CH₃ or
W = H, X = CH₃; Y = Cl, Z = CH₃ or
W =H; X = Br; Y = CH₃; Z = Cl or
W = H; X = Br; Y = CH₃; Z = CH₃ or
W = H; X = Cl; Y = Br; Z = CH₃ or
W = H; X = Cl; Y = Cl; Z = CH₃ or
W = H; X = CH₃; Y = CH₃; Z = Br or
W = H; X = Cl; Y, Z = -O-CF₂-O- or
W = H; X = Br, Y = CH₃; Z = Br or
W = H; X = CH₃; Y = H; Z = CH₃ or
W = H; X = Cl; Y = H; Z = NO₂ or
W = H; X = Br, Y = H; Z = OCH₃,
G = H,
| **A** | **B** |
|---|---|
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -(CH₂)₂-S-(CH₂)₂- | |
| -CH₂-O-(CH₂)₃- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHOi-C₃H₇-(CH₂)₂- | |
| -(CH₂)₂-C(CH₃)₂-(CH₂)₂- | |
| -CH₂-(CHCH₃)₂-(CH₂)₂- | |

2. Compounds of the formula (I) 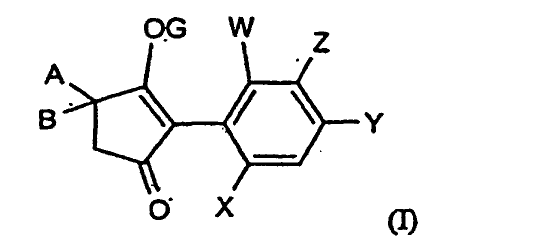 in which the substituents are as defined in the table

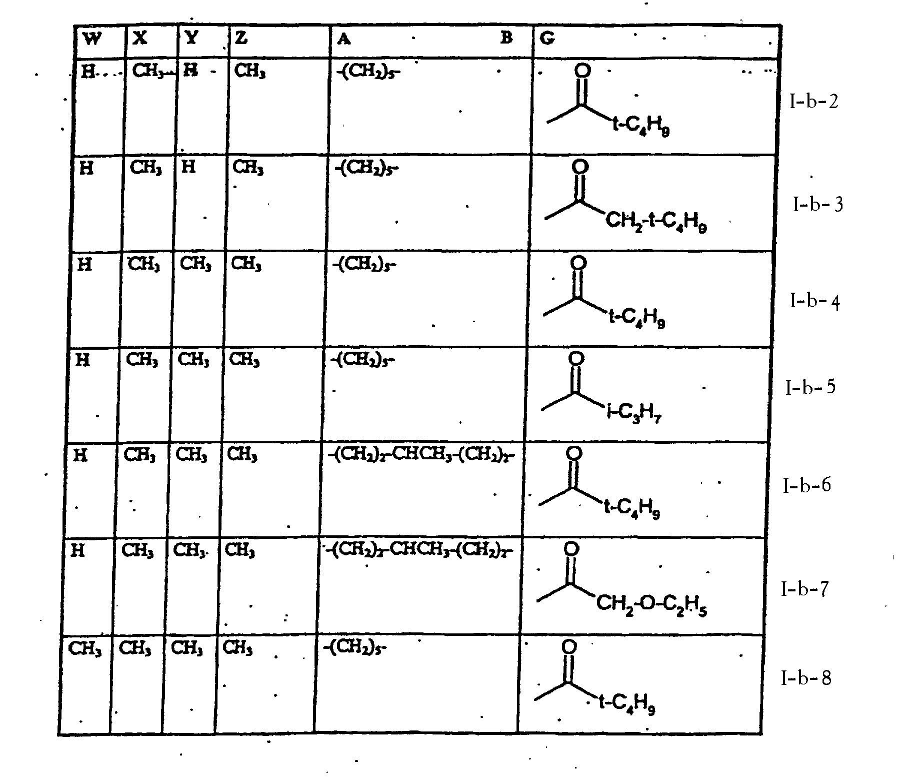

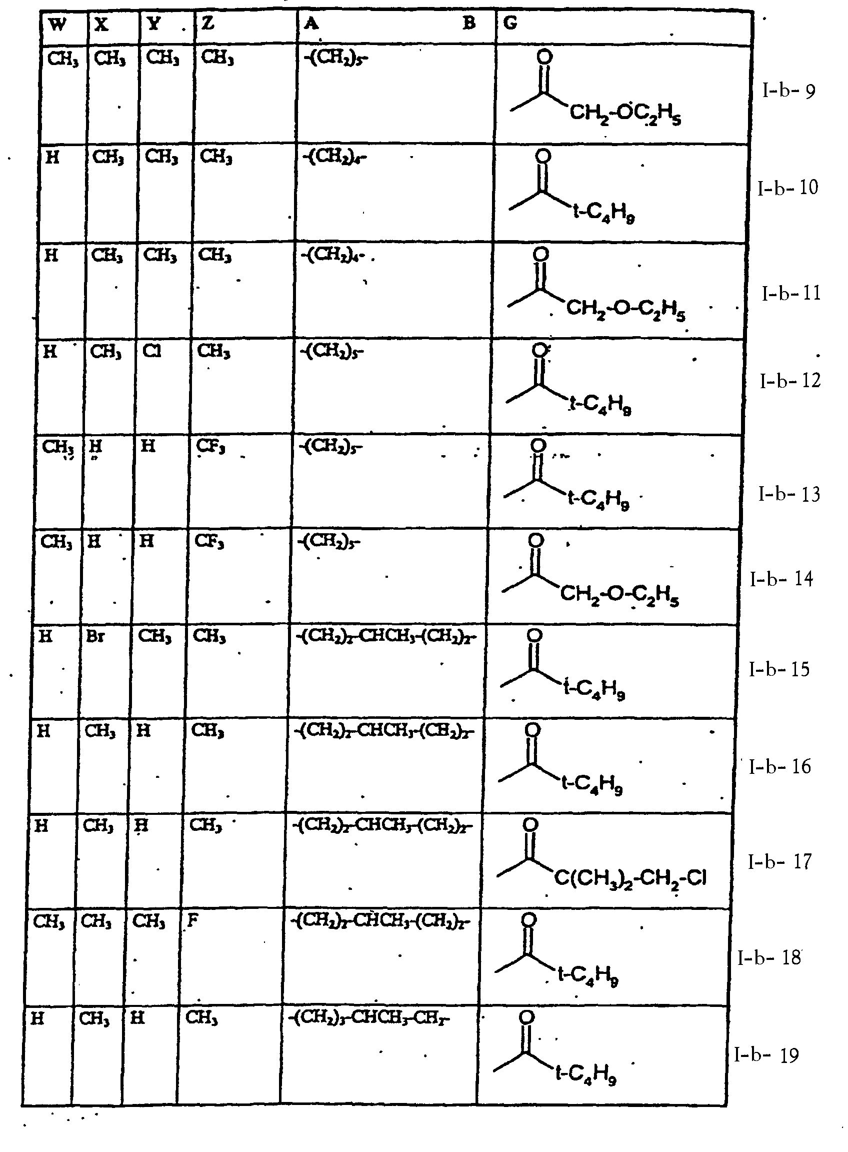

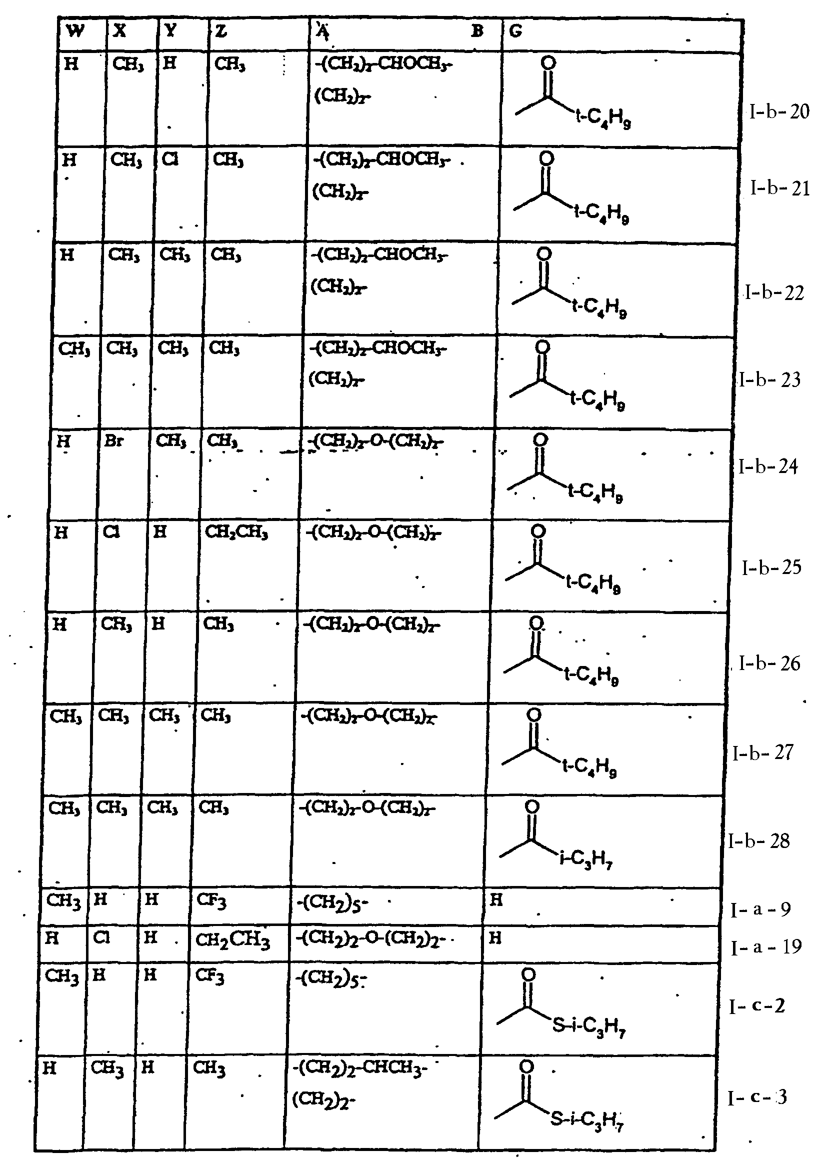

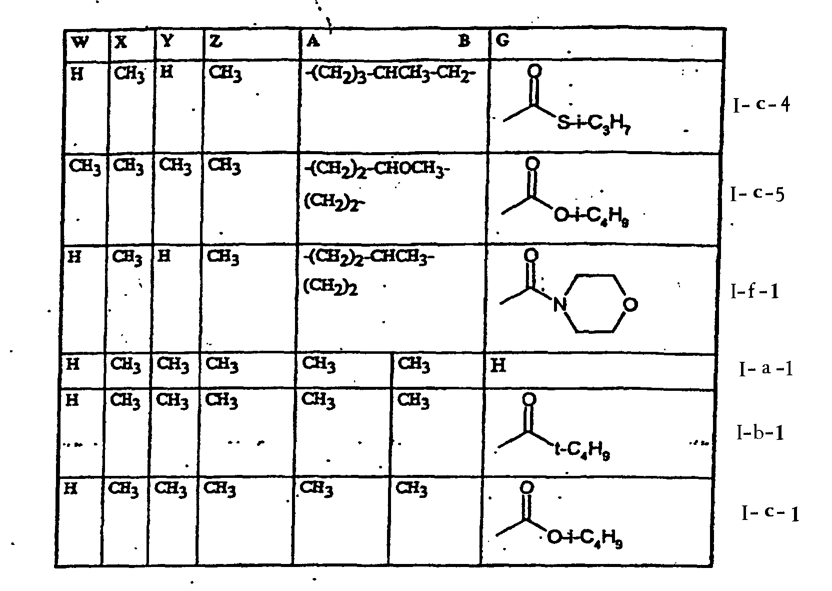

3. Compounds of the formula (II) 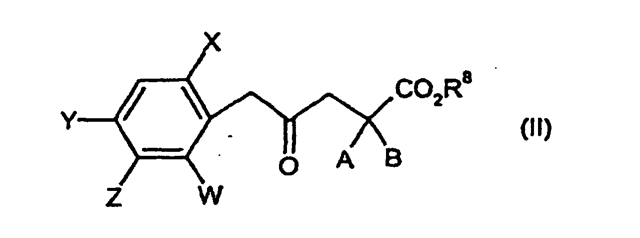 in which the substituents are as defined in the table
| **W** | **X** | **Y** | **Z** | **A** | **B** | **R8** | |
|---|---|---|---|---|---|---|---|
| H | CH₃ | H | CH₃ | -(CH₂)₅- | | CH₃ | II-2 |
| H | CH₃ | CH₃ | CH₃ | -(CH₂)₅- | | CH₃ | II-3 |
| CH₃ | CH₃ | CH₃ | CH₃ | -(CH₂)₅- | | CH₃ | II-4 |
| H | CH₃ | CH₃ | CH₃ | -(CH₂)₂-CHCH₃-(CH₂)₂ | | CH₃ | II-5 |
| H | CH₃ | H | CH₃ | -(CH₂)₄- | | CH₃ | II-6 |
| H | CH₃ | CH₃ | CH₃ | -(CH₂)₄- | | CH₃ | II-7 |
| H | CH₃ | Cl | CH₃ | -(CH₂)₅- | | CH₃ | II-8 |
| CH₃ | H | H | CF₃ | -(CH₂)₅- | | CH₃ | II-9 |
| H | Br | CH₃ | CH₃ | -(CH₂)₂-CHCH₃-(CH₂)₂ | | CH₃ | II-10 |
| H | CH₃ | H | CH₃ | -(CH₂)₂-CHCH₃-(CH₂)₂- | | CH₃ | II-11 |
| CH₃ | CH₃ | CH₃ | F | -(CH₂)₂CHCH₃-(CH₂)₂- | | CH₃ | II-12 |
| H | CH₃ | H | CH₃ | -(CH₂)₃-CHCH₃-CH₂- | | CH₃ | II-13 |
| H | CH₃ | H | CH₃ | -(CH₂)₂-CHOCH₃-(CH₂)₂- | | CH₃ | II-14 |
| H | CH₃ | Cl | CH₃ | -(CH₂)₂-CHOCH₃-(CH₂)₂- | | CH₃ | II-15 |
| H | CH₃ | CH₃ | CH₃ | -(CH₂)₂-CHOCH₃-(CH₂)₂- | | CH₃ | II-16 |
| CH₃ | CH₃ | CH₃ | CH₃ | -(CH₂)₂-CHCCH₃-(CH₂)₂- | | CH₃ | II-17 |
| H | Br | CH₃ | CH₃ | -(CH₂)₂-O-(CH₂)₂- | | CH₃ | II-18 |
| H | Cl | H | CH₂CH₃ | -(CH₂)₂-O-(CH₂)₂- | | CH₃ | II-19 |
| H | CH₃ | H | CH₃ | -(CH₂)₂-O-(CH₂)₂- | | CH₃ | II-20 |
| CH₃ | CH₃ | CH₃ | CH₃ | -(CH₂)₂-O-(CH₂)₂- | | CH₃ | II-21 |
| H | CH₃ | CH₃ | CH₃ | CH₃ | CH₃ | CH₃ | II-1 |

4. Compounds of the formula (XIII) 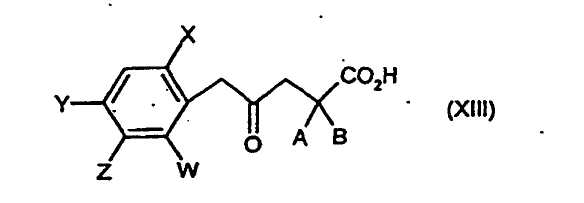 in which the substituents are as defined in the table
| **W** | **X** | **Y** | **Z** | **A** | **B** | |
|---|---|---|---|---|---|---|
| H | CH₃ | H | CH₃ | -(CH₂)₅- | | XIII-2 |
| H | CH₃ | CH₃ | CH₃ | -(CH₂)₅- | | XIII-3 |
| CH₃ | CH₃ | CH₃ | CH₃ | -(CH₂)₅- | | XIII-4 |
| H | CH₃ | CH₃ | CH₃ | -(CH₂)₂-CHCH₃-(CH₂)₂ | | XIII-5 |
| H | CH₃ | H | CH₃ | -(CH₂)₄- | | XIII-6 |
| H | CH₃ | CH₃ | CH₃ | -(CH₂)₄- | | XIII-7 |
| H | CH₃ | Cl | CH₃ | -(CH₂)₅- | | XIII-8 |
| CH₃ | H | H | CF₃ | -(CH₂)₅- | | XIII-9 |
| H | Br | CH₃ | CH₃ | -(CH₂)₂-CHCH₃-(CH₂)₂ | | XIII-10 |
| H | CH₃ | H | CH₃ | -(CH₂)₂-CHCH₃-(CH₂)₂- | | XIII-11 |
| CH₃ | CH₃ | CH₃ | F | -(CH₂)₂CHCH₃-(CH₂)₂- | | XIII-12 |
| H | CH₃ | H | CH₃ | -(CH₂)₃-CHCH₃-CH₂- | | XIII-13 |
| H | CH₃ | H | CH₃ | -(CH₂)₂-CHOCH₃-(CH₂)₂- | | XIII-14 |
| H | CH₃ | Cl | CH₃ | -(CH₂)₂-CHOCH₃-(CH₂)₂- | | XIII-15 |
| H | CH₃ | CH₃ | CH₃ | -(CH₂)₂-CHOCH₃-(CH₂)₂- | | XIII-16 |
| CH₃ | CH₃ | CH₃ | CH₃ | -(CH₂)₂-CHCCH₃-(CH₂)₂- | | XIII-17 |
| H | Br | CH₃ | CH₃ | -(CH₂)₂-O-(CH₂)₂- | | XIII-18 |
| H | Cl | H | CH₂CH₃ | -(CH₂)₂-O-(CH₂)₂- | | XIII-19 |
| H | CH₃ | H | CH₃ | -(CH₂)₂-O-(CH₂)₂- | | XIII-20 |
| CH₃ | CH₃ | CH₃ | CH₃ | -(CH₂)₂-O-(CH₂)₂- | | XIII-21 |
| H | CH₃ | CH₃ | CH₃ | CH₃ | CH₃ | XIII-1 |

5. Pesticides or herbicides, **characterized in that** they contain a compound of the formula (I) according to Claims 1 and 2.

6. Use of compounds of the formula (I) according to Claims 1 and 2 for controlling pests and weeds.

7. Method for controlling pests and weeds, **characterized in that** compounds of the formula (I) according to Claims 1 and 2 are allowed to act on pests or weeds and/or their habitat.

8. Process for preparing pesticides and herbicides, **characterized in that** compounds of the formula (I) according to Claims 1 and 2 are mixed with extenders and/or surfactants.

9. Process for preparing compounds of the formula (I) according to Claims 1 and 2, **characterized in that**
(A)compounds of the formula (II) 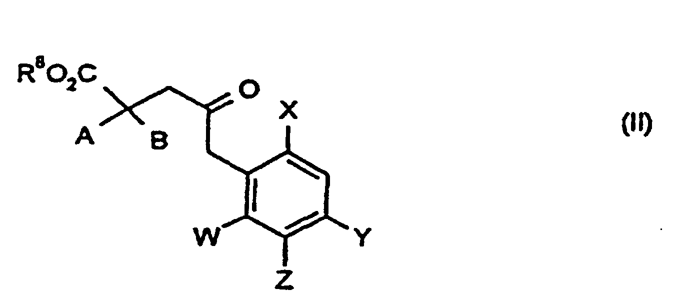 in which
A, B, W, X, Y and Z are each as defined above and
R⁸ represents alkyl,
are cyclized intramolecularly, if appropriate in the presence of a diluent and in the presence of a base, and the resulting compounds of the formula (Ia) 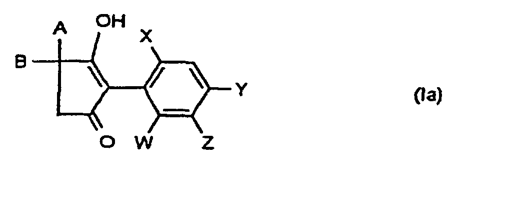 in which
A, B, W, X, Y and Z are each as defined in Claim 1
are subsequently, if appropriate, reacted
(B)α) with acyl halides of the formula (III) 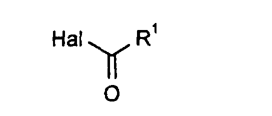 in which
R¹ is t-C₄H₉, CH₂-t-C₄H₉, i-C₃H₇, CH₂OC₂H₅ or C(CH₃)₂CH₂Cl and
Hal represents halogen,
or
β) with carboxylic anhydrides of the formula (IV)
R¹-CO-O-CO-R¹ (IV)
in which
R¹ is as defined above,
if appropriate in the presence of a diluent and if appropriate in the presence of an acid binder;
or
(C)with chloroformic esters or chloroformic thioesters of the formula (V)
R²-M-CO-Cl (V)
in which
R² is i-C₃H₇ or i-C₄H₉ and M is oxygen or sulphur,
if appropriate in the presence of a diluent and if appropriate in the presence of an acid binder;
or
(G)
β) with carbamoyl chlorides or thiocarbamoyl chlorides of the formula (XII) 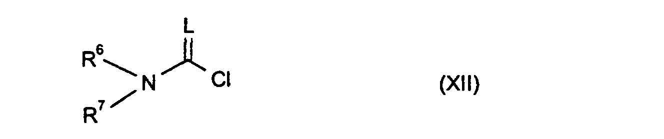 in which
L, is oxygen and
R⁶ and R⁷ together with the N-atom to which they are attached represent the group 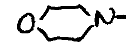 if appropriate in the presence of a diluent and if appropriate in the presence of an acid binder.

## Revendications

1. Composés de formule (I) : 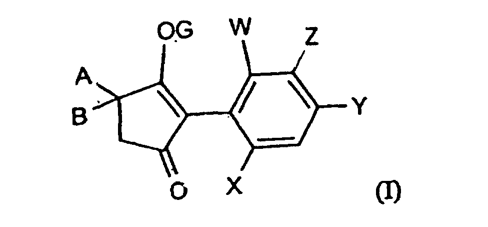 dans laquelle les substituants ont les définitions indiquées ci-après :
W = CH₃ ; X = CH₃ ; Y = CH₃ ; Z = CH₃ ou
W = CH₃ ; X = CH₃ ; Y = H ; Z = Cl ou
W = CH₃ ; X = CH₃ ; Y = CH₃ ; Z = F ou
W = CH₃ ; X = CH₃ ; Y = CH₃ ; Z = Cl ou
W = CH₃ ; X = CH₃ ; Y = CH₃ ; Z = Br ou
W = CH₃ ; X = CH₃ ; Y = H ; Z = Br ou
W = Cl ; X = Cl ; Y = H ; Z = Br ou
W = Br ; X = Br ; Y, Z = - (CH₂)₃- ou
W = CH₃ ; X = OCH₃ ; Y = H ; Z = Br ou
W = CH₃ ; X = CH₃ ; Y = CH₃ ; Z = CH₃ ou
W = Cl ; X = Cl ; Y = Cl ; Z = CH₃ ou
W = Br ; X = Br ; Y = Br ; Z = CH₃ ou
W = H ; X = Cl ; Y = Cl ; Z = Cl ou
W = H ; X = CH₃ ; Y = CH₃ ; Z = CH₃ ou
W = H ; X = CH₃ ; Y = Cl ; Z = CH₃ ou
W = H ; X = Br ; Y = CH₃ ; Z = Cl ou
W = H ; X = Br ; Y = CH₃ ; Z = CH₃ ou
W = H ; X = Cl ; Y = Br ; Z = CH₃ ou
W = H ; X = Cl ; Y = Cl ; Z = CH₃ ou
W = H ; X = CH₃ ; Y = CH₃ ; Z = Br ou
W = H ; X = Cl ; Y,Z = -O-CF₂-O- ou
W = H ; X = Br ; Y = CH₃ ; Z = Br ou
W = H ; X = CH₃ ; Y = H ; Z = CH₃ ou
W = H ; X = Cl ; Y = H ; Z = NO₂ ou
W = H ; X = Br ; Y = H ; Z = OCH₃ ou
G = H,
| **A** | **B** |
|---|---|
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -(CH₂)₂-S-(CH₂)₂- | |
| -CH₂-O-(CH₂)₃- | |
| -CH₂-CHCH₃-(CH₂)₃- | |
| -(CH₂)₂-CHCH₃-(CH₂)₂- | |
| -(CH₂)₂-CHC₂H₅-(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHOi-C₃H₇-(CH₂)₂- | |
| -(CH₂)₂-C(CH₃)₂-(CH₂)₂- | |
| -CH₂-(CHCH₃)₂-(CH₂)₂- | |

2. Composés de formule (I) 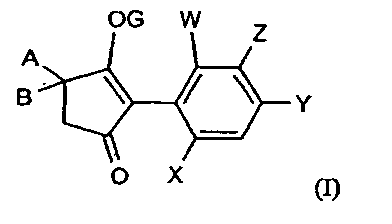 dans laquelle les substituants ont les définitions indiquées dans le tableau

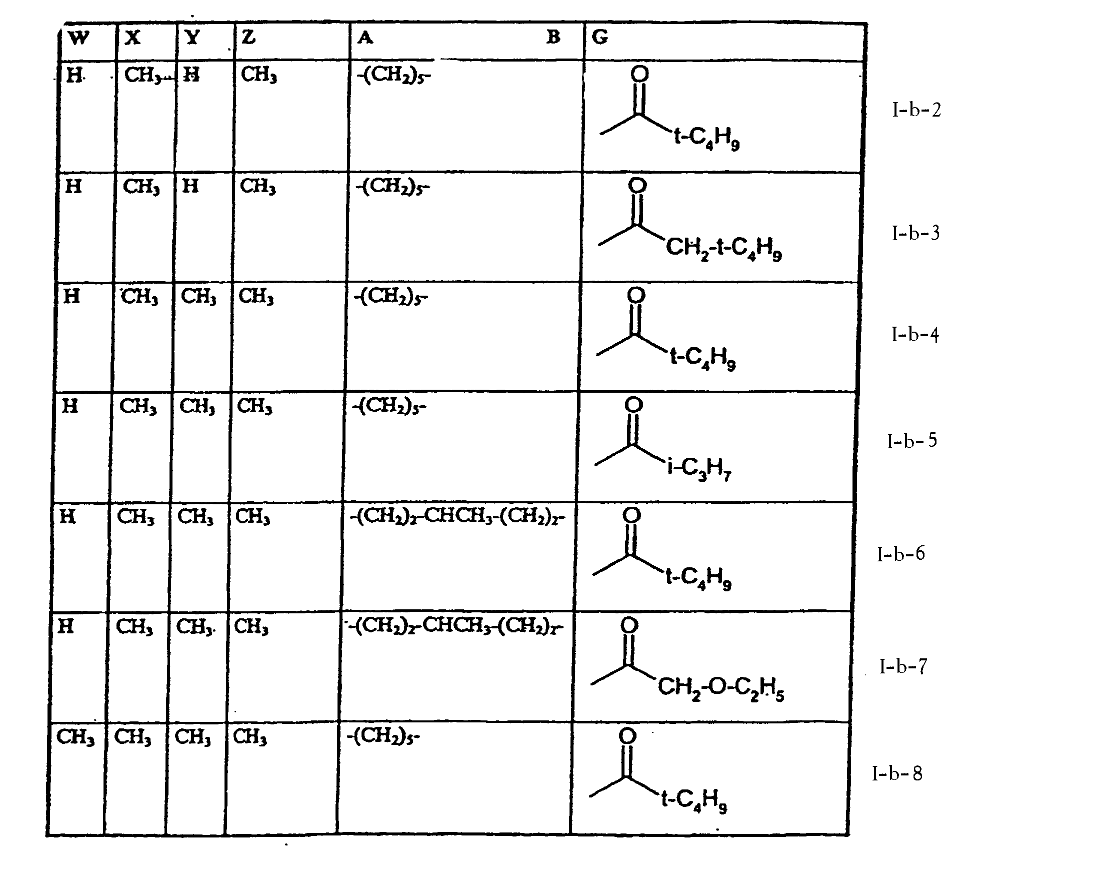

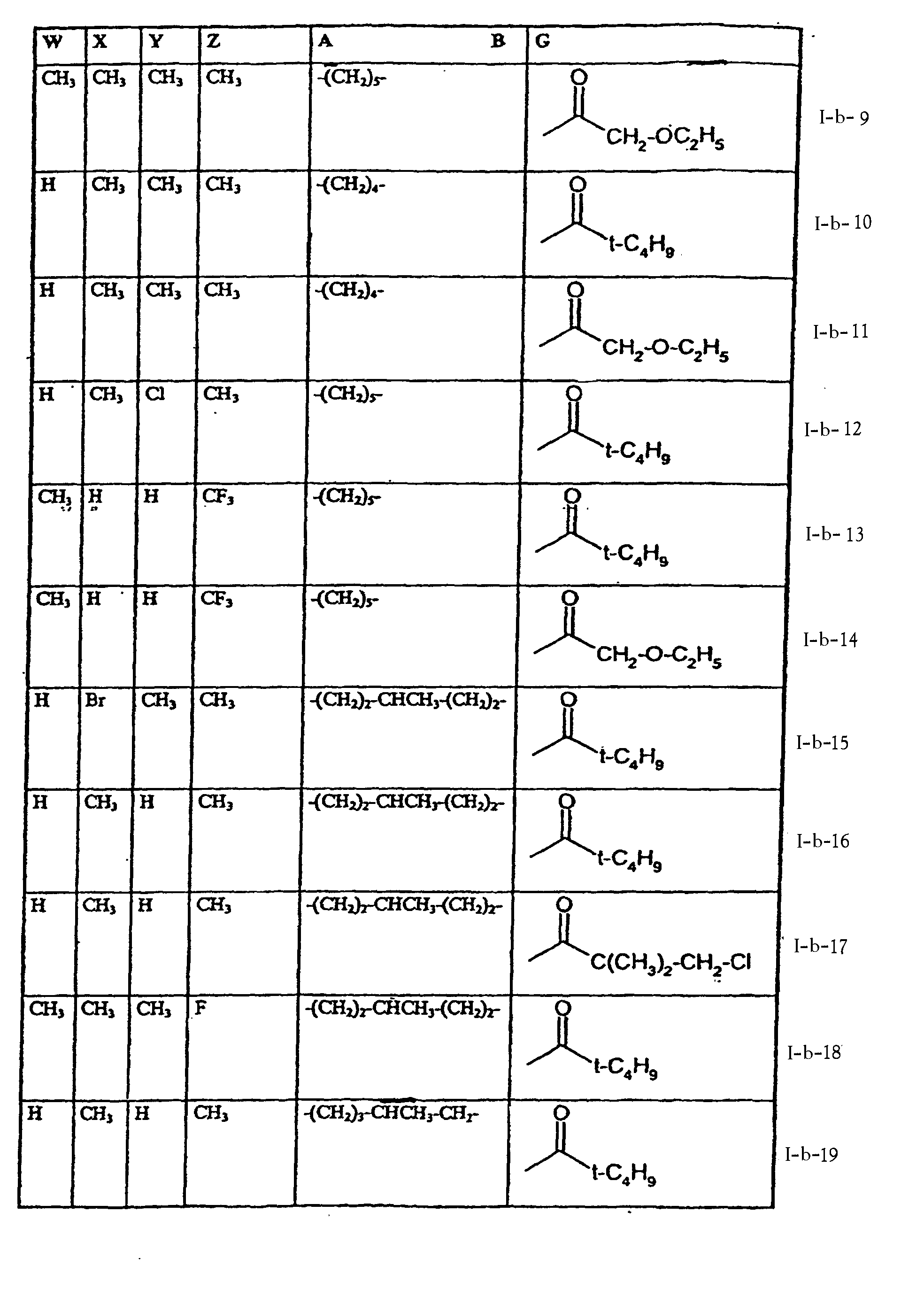

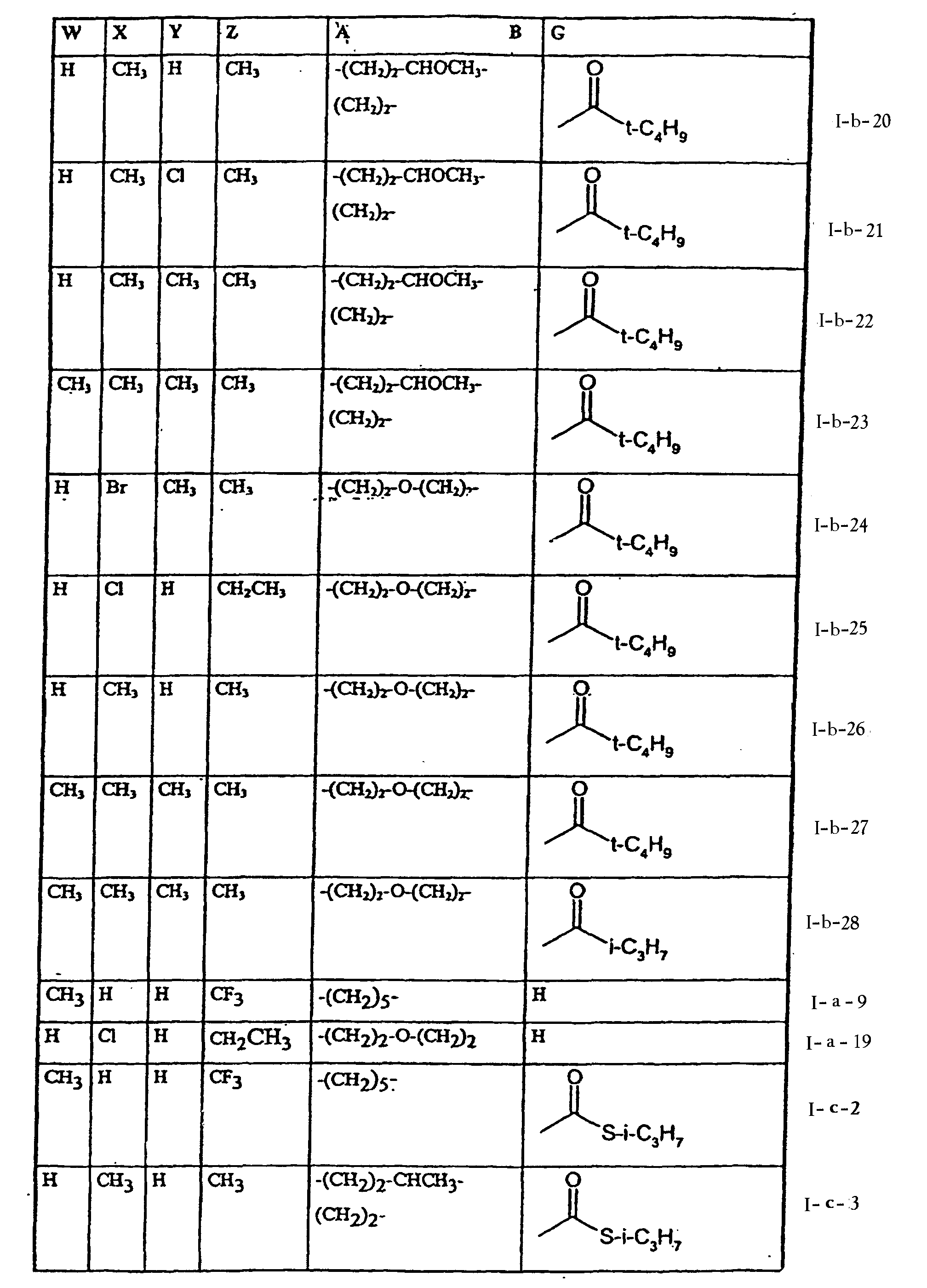

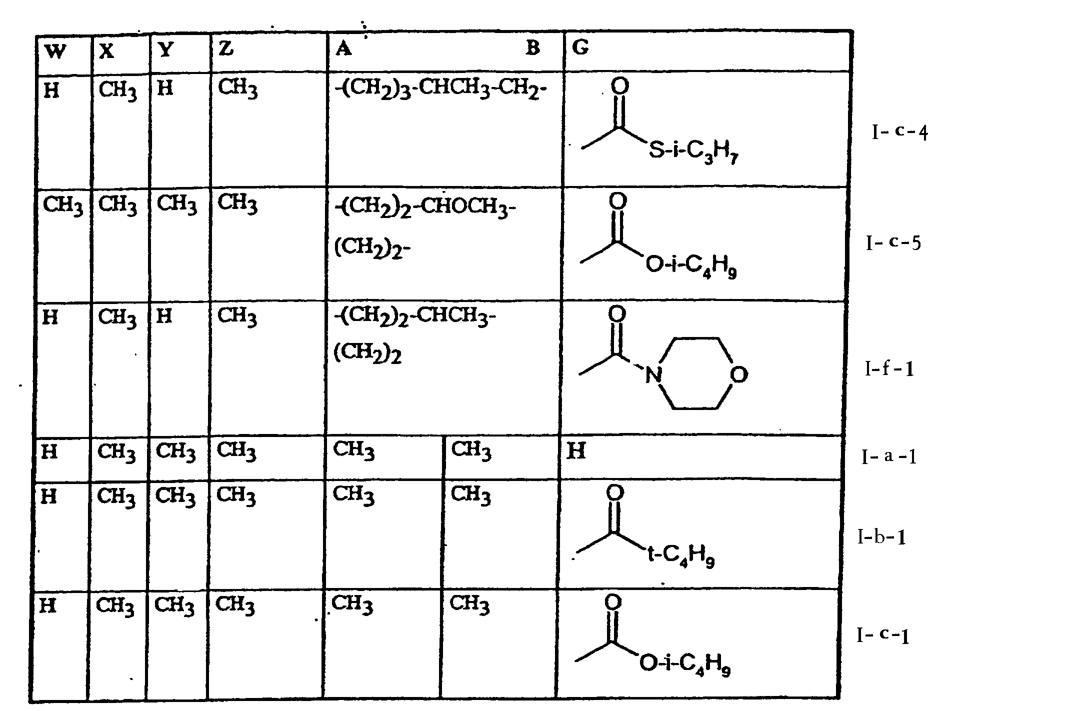

3. Composés de formule (II) 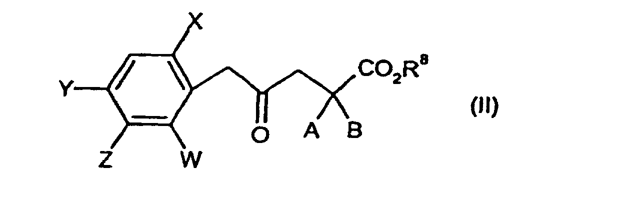 dans laquelle les substituants ont les définitions indiquées dans le tableau
| **W** | **X** | **Y** | **Z** | **A** | **B** | **R8** | |
|---|---|---|---|---|---|---|---|
| H | CH₃ | H | CH₃ | -(CH₂)₅- | | CH₃ | II-2 |
| H | CH₃ | CH₃ | CH₃ | -(CH₂)₅- | | CH₃ | II-3 |
| CH₃ | CH₃ | CH₃ | CH₃ | -(CH₂)₅- | | CH₃ | II-4 |
| H | CH₃ | CH₃ | CH₃ | -(CH₂)₂-CHCH₃-(CH₂)₂- | | CH₃ | II-5 |
| H | CH₃ | H | CH₃ | -(CH₂)₄- | | CH₃ | II-6 |
| H | CH₃ | CH₃ | CH₃ | -(CH₂)₄- | | CH₃ | II-7 |
| H | CH₃ | Cl | CH₃ | -(CH₂)₅- | | CH₃ | II-8 |
| CH₃ | H | H | CF₃ | -(CH₂)₅- | | CH₃ | II-9 |
| H | Br | CH₃ | CH₃ | -(CH₂)₂-CHCH₃-(CH₂)₂ | | CH₃ | II-10 |
| H | CH₃ | H | CH₃ | -(CH₂)₂-CHCH₃-(CH₂)₂- | | CH₃ | II-11 |
| CH₃ | CH₃ | CH₃ | F | -(CH₂)₂CHCH₃-(CH₂)₂- | | CH₃ | II-12 |
| H | CH₃ | H | CH₃ | -(CH₂)₃-CHCH₃-CH₂- | | CH₃ | II-13 |
| H | CH₃ | H | CH₃ | -(CH₂)₂-CHOCH₃-(CH₂)₂- | | CH₃ | II-14 |
| H | CH₃ | Cl | CH₃ | -(CH₂)₂-CHOCH₃-(CH₂)₂- | | CH₃ | II-15 |
| H | CH₃ | CH₃ | CH₃ | -(CH₂)₂-CHOCH₃-(CH₂)₂- | | CH₃ | II-16 |
| CH₃ | CH₃ | CH₃ | CH₃ | -(CH₂)₂-CHCCH₃-(CH₂)₂- | | CH₃ | II-17 |
| H | Br | CH₃ | CH₃ | -(CH₂)₂-O-(CH₂)₂- | | CH₃ | II-18 |
| H | Cl | H | CH₂CH₃ | -(CH₂)₂-O-(CH₂)₂- | | CH₃ | II-19 |
| H | CH₃ | H | CH₃ | -(CH₂)₂-O-(CH₂)₂- | | CH₃ | II-20 |
| CH₃ | CH₃ | CH₃ | CH₃ | -(CH₂)₂-O-(CH₂)₂- | | CH₃ | II-21 |
| H | CH₃ | CH₃ | CH₃ | CH₃ | CH₃ | CH₃ | II-1 |

4. Composés de formule (XIII) 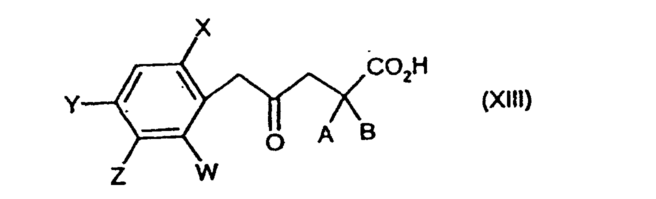 dans laquelle les substituants ont les définitions indiquées dans le tableau
| **W** | **X** | **Y** | **Z** | **A** | **B** | |
|---|---|---|---|---|---|---|
| H | CH₃ | H | CH₃ | -(CH₂)₅- | | XIII-2 |
| H | CH₃ | CH₃ | CH₃ | -(CH₂)₅- | | XIII-3 |
| CH₃ | CH₃ | CH₃ | CH₃ | -(CH₂)₅- | | XIII-4 |
| H | CH₃ | CH₃ | CH₃ | -(CH₂)₂-CHCH₃-(CH₂)₂- | | XIII-5 |
| H | CH₃ | H | CH₃ | -(CH₂)₄- | | XIII-6 |
| H | CH₃ | CH₃ | CH₃ | -(CH₂)₄- | | XIII-7 |
| H | CH₃ | Cl | CH₃ | -(CH₂)₅- | | XIII-8 |
| CH₃ | H | H | CF₃ | -(CH₂)₅- | | XIII-9 |
| H | Br | CH₃ | CH₃ | -(CH₂)₂-CHCH₃-(CH₂)₂ | | XIII-10 |
| H | CH₃ | H | CH₃ | -(CH₂)₂-CHCH₃-(CH₂)₂- | | XIII-11 |
| CH₃ | CH₃ | CH₃ | F | -(CH₂)₂CHCH₃-(CH₂)₂- | | XIII-12 |
| H | CH₃ | H | CH₃ | -(CH₂)₃-CHCH₃-CH₂- | | XIII-13 |
| H | CH₃ | H | CH₃ | -(CH₂)₂-CHOCH₃-(CH₂)₂- | | XIII-14 |
| H | CH₃ | Cl | CH₃ | -(CH₂)₂-CHOCH₃-(CH₂)₂- | | XIII-15 |
| H | CH₃ | CH₃ | CH₃ | -(CH₂)₂-CHOCH₃-(CH₂)₂- | | XIII-16 |
| CH₃ | CH₃ | CH₃ | CH₃ | -(CH₂)₂-CHCCH₃-(CH₂)₂- | | XIII-17 |
| H | Br | CH₃ | CH₃ | -(CH₂)₂-O-(CH₂)₂- | | XIII-18 |
| H | Cl | H | CH₂CH₃ | -(CH₂)₂-O-(CH₂)₂- | | XIII-19 |
| H | CH₃ | H | CH₃ | -(CH₂)₂-O-(CH₂)₂- | | XIII-20 |
| CH₃ | CH₃ | CH₃ | CH₃ | -(CH₂)₂-O-(CH₂)₂- | | XIII-21 |
| H | CH₃ | CH₃ | CH₃ | CH₃ | CH₃ | XIII-1 |

5. Compositions pesticides ou compositions herbicides, **caractérisées par** une teneur en un composé de formule (I) suivant les revendications 1 et 2.

6. Utilisation de composés de formule (I) suivant les revendications 1 et 2 pour combattre des parasites et des mauvaises herbes.

7. Procédés de lutte contre des parasites et des mauvaises herbes, **caractérisés en ce qu'**on fait agir des composés de formule (I) suivant les revendications 1 et 2 sur les parasites ou les mauvaises herbes et/ou sur leur milieu.

8. Procédés de préparation de compositions pesticides et de compositions herbicides, **caractérisés en ce qu'**on mélange des composés de formule (I) suivant la revendication 1 et 2 avec des diluants et/ou des agents tensioactifs.

9. Procédé de production de composés de formule (I) suivant les revendications 1 et 2, **caractérisé en ce que** :
(A) on effectue la cyclisation intramoléculaire de composés de formule (II) 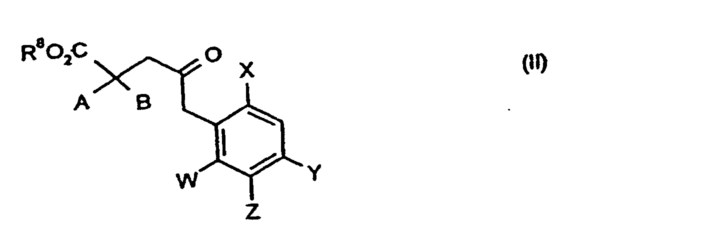 dans laquelle
A, B, W, X, Y, Z ont la définition indiquée ci-dessus et
R⁸ est un reste alkyle,
éventuellement en présence d'un diluant et en présence d'une base et, le cas échant, on fait réagir ensuite les composés ainsi obtenus de formule (Ia) 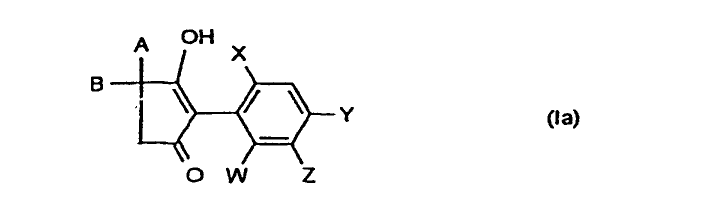 dans laquelle
A, B, W, X, Y et Z ont la définition indiquée dans la revendication 1,
(B) α) avec des halogénures d'acide de formule (III) 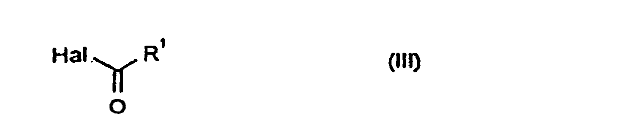 dans laquelle
R¹ est t-C₄H₉, CH₂-t-C₄H₉, i-C₃H₇, CH₂OC₂H₅ ou C(CH₃)₂CH₂Cl et
Hal est un halogène,
ou bien
β) avec des anhydrides d'acide carboxylique de formule (IV)
R¹-CO-O-CO-R¹ (IV)
dans laquelle
R¹ a la définition indiquée ci-dessus,
le cas échéant en présence d'un diluant et en présence éventuelle d'un accepteur d'acide ;
ou bien
(C) avec des esters d'acide chloroformique ou des thioesters d'acide chloroformique de formule (V)
R²-M-CO-Cl (V)
dans laquelle
R² est i-C₃H₇ ou i-C₄H₉ et M est O ou S,
éventuellement en présence d'un diluant et, le cas échéant, en présence d'un accepteur d'acide ;
ou bien
(G)
β) on les fait réagir avec des chlorures d'acide carbamique ou des chlorures d'acide thiocarbamique de formule (XII) 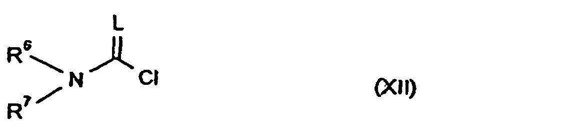 dans laquelle L est O et
R⁶ et R⁷ forment ensemble le groupe 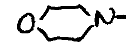 éventuellement en présence d'un diluant et, le cas échant, en présence d'un accepteur d'acide.
